(19) Europäisches Patentamt
European Patent Office
Office européen des brevets

(11) EP 2 253 665 B1

(12) **EUROPÄISCHE PATENTSCHRIFT**

(45) Veröffentlichungstag und Bekanntmachung des Hinweises auf die Patenterteilung:
**05.09.2018 Patentblatt 2018/36**

(21) Anmeldenummer: **10160420.5**

(22) Anmeldetag: **20.04.2010**

(51) Int Cl.:
***C08L 33/02*** *(2006.01)*
***A61K 9/19*** *(2006.01)*
***A61K 9/00*** *(2006.01)*
***A61L 15/22*** *(2006.01)*
***A61L 15/60*** *(2006.01)*

(54) **Gefriergetrocknete Zusammensetzung**

Freeze-dried composition

Composition lyophilisée

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **22.04.2009 EP 09158478**

(43) Veröffentlichungstag der Anmeldung:
**24.11.2010 Patentblatt 2010/47**

(73) Patentinhaber: **MedSkin Solutions Dr. Suwelack AG**
**48721 Billerbeck (DE)**

(72) Erfinder:
- **Wiesweg, Karin**
  **48653, Coesfeld (DE)**
- **Elsinghorst, Claudia**
  **48727, Billerbeck (DE)**
- **MALESSA, Ralf**
  **45134, Essen (DE)**

(74) Vertreter: **CH Kilger Anwaltspartnerschaft mbB**
**Fasanenstrasse 29**
**10719 Berlin (DE)**

(56) Entgegenhaltungen:
**WO-A-00/22083**
**WO-A-01/13967**
**WO-A-01/28600**
**WO-A-01/82886**
**WO-A-94/01468**
**WO-A-99/65538**
**WO-A-03/051412**
**WO-A-03/068843**
**WO-A-2005/123034**
**WO-A-2007/122232**
**WO-A-2008/070270**
**GB-A- 2 080 814**
**GB-A- 2 431 104**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 253 665 B1

## Beschreibung

Die Erfindung betrifft gefriergetrocknete Zusammensetzungen enthaltend

a) mindestens ein Polymer auf Basis von Polyacrylsäuren und Salzen davon,
b) mindestens ein natürliches Polymer, ausgewählt aus der Gruppe der Alginate, b) mindestens ein weiteres natürliches Polymer ausgewählt aus der Gruppe des Carrageenans und seiner Derivate, der Hvaluraonsäure und ihrer Derivate und/oder des Collagens und seiner Derivate,
d) gegebenenfalls mindestens ein weiteres von a) b) und c) verschiedenes Polymer sowie
e) gegebenenfalls einen oder mehrere Wirk- und/oder Hilfsstoffe.

Weiterhin betrifft die Erfindung ein Verfahren zur Herstellung solcher gefriergetrockneten Zusammensetzungen, die Kombination solcher gefriergetrockneten Zusammensetzungen in Kit-of-parts Anordnungen zusammen mit wässrigen Lösungen sowie die gefriergetrockneten Zusammensetzungen und Verwendung der der Kit-of-parts Kombinationen zur kosmetischen und pharmazeutischen Anwendung, insbesondere zur Verwendung als kosmetische Maske oder als Wundauflage und zur Herstellung von pharmazeutischen Mitteln zur Behandlung von dermalen Wunden wie insbesondere chronischen Wunden, Ulcus cruris oder Dekubitus.

Es sind diverse Mittel zur kosmetischen und therapeutischen Behandlung des menschlichen und/oder tierischen Körpers in den unterschiedlichsten Darreichungs- und Applikationsformen bekannt.
Eine wichtige Rolle, insbesondere im pharmazeutischen Bereich der Wundversorgung, aber zunehmend auch im Bereich der kosmetischen Anwendungen spielen hierbei Zusammensetzungen und Mittel in Form von festen, trockenen Zubereitungen mit einer Ausgestaltung als Schwämme, Sheets, Matrices, Auflagen, Pads, Blätter, Masken, Lagen oder anderen flächigen Formen sowie solche in Form großformatiger Formkörper. Solche Ausführungsformen eignen sich besonders zur äußerlichen und flächigen Behandlung und Pflege der Haut sowie insbesondere zur Versorgung von flächigen Hautverletzungen oder
Wunden. Je nach gewünschtem Behandlungsziel oder gewähltem Einsatzgebiet solcher Auflagen sind besondere, mitunter sehr spezifische Material- und Funktions-Anforderungen an solche Zusammensetzungen, insbesondere an ihre chemische Zusammensetzung sowie ihre physikalische oder biologischchemische Wirk- und Funktionsweise, zu stellen. Speziell bei der äußerlichen dermalen Behandlung sind die komplexen biochemischen Wechselwirkungen und Funktionsweisen mit dem Organ "Haut" zu beachten.

Dabei sind nahezu gleichermaßen die Pflege und der Schutz der Haut z. B. durch eine kosmetische Behandlung sowie die Wiederherstellung, Heilung oder Linderung von Funktionsstörungen oder Verletzungen der Haut durch eine therapeutische Behandlung von Bedeutung.

Die Pflege und der vorbeugende Schutz durch eine kosmetische Behandlung kann insbesondere durch die Auf- und Einbringung von Wirk-, Nähr- und/oder Pflegestoffen erzielt werden, aber auch durch Unterstützung oder Verbesserung der physikalischen und mechanischen Schutz- und/oder Barriere-Eigenschaften wie Elastizität, Glätte/Rauhigkeit, Trockenheit oder biochemisches Gleichgewicht der Haut. Hier stellt insbesondere die Unterstützung, der Schutz, die Regulierung sowie Verbesserung des Feuchtigkeits- und Fettgehaltes, insbesondere des sogenannten "Natural" Moisturizing Factors" (NMF) sowie der Barrierefunktion der Haut ein wichtiges Behandlungselement dar.

Bei einer Verletzung oder Beeinträchtigung der Haut oder einer ihrer zentralen Funktionen ist eine Behandlung von besonderer Bedeutung, die eine lindernde, heilende oder wiederherstellende Wirkung mit sich bringt. Eine solche therapeutische Behandlung kann ebenfalls durch Zuführung bestimmter positiv wirkender Aktiv-, Heil- oder Wirkstoffe erfolgen oder durch geeignete unterstützende physikalische oder biochemische Methoden, die die Selbstheilung unterstützen und günstig beeinflussen. Die Art und der Umfang einer solchen therapeutischen Behandlung ist dabei insbesondere von der Art der Verletzung oder Funktionsstörung abhängig und speziell auf die betroffenen Hautschichten abzustimmen. Hier ist insbesondere im Bereich der Wundversorgung das sogenannte WundexsudatManagement, das Wunddebridement oder die Beeinflussung und Regulierung des Wundklimas zu nennen.

Für beide Bereiche, den pflegenden kosmetischen sowie den therapeutischen Bereich der Hautbehandlung, ist die Verwendung von festen, trockenen, absorbierenden bzw. hydratisierbaren Zubereitungsformen, insbesondere in Form von flächigen Sheets, Auflagen oder Masken prinzipiell besonders geeignet und auch bereits weit verbreitet. Dabei sind besonders solche Zubereitungen von Interesse, die neben der Wirkstoff-Applikation auch an sich bereits eine die Haut hydratisierende Wirkung aufweisen. Dies ist gleichermaßen relevant für die kosmetische als auch therapeutische Hautbehandlung.

Zu diesem Zweck sind insbesondere Zubereitungen auf Basis von hydrophilen, quellbaren Hydrokolloiden oder Polymeren bekannt und genutzt. Speziell aus dem therapeutischen Bereich der Wundbehandlung sind besonders Hydrogele als Wundbehandlungsmittel bekannt. Hydrogele zeichnen sich dabei insbesondere durch einen hohen Wassergehalt bzw. eine hohe Flüssigkeitsaufnahme- und -speicherkapazität aus, womit sie besonders für die feuchte Wundbehandlung besonders geeignet sind. Besonders geeignete Zusammensetzungen stellen Hydrogele auf Basis von

Polyacrylaten und ihren Derivaten bzw. solche auf Basis von Polyacrylsäuren und deren Salzen dar. Insbesondere synthetische Acrylsäurepolymere der sogenannten Gruppe der "Carbomere", zeichnen sich durch besonders gute Wasseraufnahmefähigkeit aus. Der Begriff "Carbomere" bezeichnet dabei gemäß USP-NF, British Pharmacopoeia, United States Adopted Names Council (USAN) sowie Cosmetic Toiletries and Fragrance Association (CTFA) die Gruppe der Carbopole. Carbomere sind außerdem unter dem Begriff "Superabsorber" bekannt.

Als "Superabsorber" (auch: superabsorbent polymers) werden üblicherweise solche Stoffe bezeichnet, die in der Lage sind, ein Vielfaches ihres Eigengewichts - bis zum 1000-fachen - an Flüssigkeiten (meist Wasser bzw. destilliertes Wasser) aufzunehmen. Bei den Carbomeren handelt es sich chemisch gesehen um komplexe Copolymere aus Acrylsäure und Natriumacrylat gegebenenfalls zusätzlichen Vernetzern und/oder Acrylsäurederivaten, wobei die chemische Zusammensetzung der Carbomere variieren kann.

Polyacrylate, ihre Derivate oder auch Carbomere, sowie Superabsorber finden sowohl im kosmetischen als auch im medizinischen Einsatzbereich ein breites Anwendungsspektrum. In der Kosmetik werden Carbomere oder Superabsorber als viskositätserhöhende Komponenten in Cremes und Lotionen eingesetzt, wie beispielsweise in der DE 10195737 beschrieben. Durch die Viskositätserhöhung im Endprodukt wirken sie zusätzlich zu den Emulgatoren einer Phasentrennung von lipophiler und hydrophiler Phase entgegen, führen zu einer reichhaltigen Textur und tragen u.a. zu einem angenehmen Hautgefühl nach der Applikation bei. Reine Hydrogel-Produkte mit einer Viskositätskomponente nur auf Polyacrylatbasis, die keine oder kaum Öle und lediglich geringe Mengen Emulgatoren enthalten, erhalten ihre Phasen-Stabilität hauptsächlich über die Viskosität der enthaltenen Polyacrylate.

Aufgrund der Fähigkeit der Polyacrylate, neben ihrer guten viskositätserhöhenden Eigenschaften außerdem extreme Mengen wässriger Flüssigkeiten aufzunehmen und unter extremer Quellung zu speichern und zu halten, werden sie besonders im therapeutischen Bereich der Wundversorgung, insbesondere in Medizinprodukten zum Exsudatmanagement eingesetzt. Hierbei wird das Polyacrylat zumeist trocken als Pulver oder Granulat eingesetzt.

Auch die Herstellung von Mikro- oder Nanopartikeln oder granulären Materialien aus Polyacrylsäure-Polymeren ist bekannt, wobei die Herstellung derartiger Partikel meist aus Mischungen der Polyacrylsäure mit weiteren stabilisierenden natürlichen oder semisynthetischen Polymeren erfolgt. So beschreibt die WO 00/22083 nanoporöse granuläre Materialien auf Basis von Acrylsäure-Carboxymethylcellulose-Lösungen und die WO 05/123034 mikropartikuläre Systeme beispielsweise auf Basis von Mischungen von Polymethacrylat- und Natriumalginatlösungen, die jeweils durch Sprühvernebelung und Gefriertrocknung der Polyacrylat-PolymerMischungen erhalten werden.

Aus der GB 2431104 ist die Verwendung von Polyacrylsäure-Polymeren zur Herstellung von Mikrogelpartikeln und deren Verwendung zur Wirkstoff-Verkapselung beispielsweise in Form einer auf eine Wundauflage aus oxidierter Baumwolle aufgebrachten Mikrogelpartikel-Suspension bekannt.

Außerdem werden Wundauflagen auf Basis von Polyacrylsäure-Polymeren häufig mit Wirkstoffen kombiniert, denen ein positiver Einfluss auf die Wundheilung nachgesagt wird. So wird z.B. eine Kombinationen mit Fibronectin in der WO 01/13967 oder eine Kombination mit fibrillärem Kollagen in der GB 2080814 oder eine Kombination mit Wachstumsfaktoren wie VEGF und PDGF in der WO 08/070270 beschrieben.

Daneben sind z.B. aus der WO 07/122232 gefriergetrocknete Zusammensetzungen für die Wundbehandlung bekannt, die durch Extrusions- oder Elektrospinning-Verfahren aus Mischungen von Gelatine mit Polyacrylsäuren erhältlich sind.

Außerdem werden Hydrogele mit Polyacrylatbestandteilen und Acrylate als Kleber oder Haftmittel im Bereich der therapeutischen Wundversorgungsmittel eingesetzt.

Sowohl in der kosmetischen als auch in der therapeutischen Hautbehandlung sind jedoch bevorzugt auch solche Zubereitungen erwünscht, die neben einer hohen Benetzungsgeschwindigkeit und einer hohen Wasseraufnahme- und -haltekapazität zusätzlich eine gute befeuchtende und die Haut hydratisierende Wirkung, also auch eine wasserabgebende Kapazität, aufweisen. Die hydratisierende Wirkung in der kosmetischen Hautbehandlung spielt eine essentielle Rolle in der Verbesserung der mechanischen und physikalischen Beschaffenheit der Haut z. B. in Hinblick auf Elastizität und Geschmeidigkeit. Im Bereich der Wundbehandlung ist eine hydratisierende Wirkung beim Einsatz in trockenen Wunden zur Verbesserung und Einstellung des Wundklimas und somit zur Unterstützung der Heilungsbedingungen in der Wunde von Bedeutung. Eine geeignete Möglichkeit, Flüssigkeitsaufnahme oder Absorption mit Hydratisierung oder Befeuchtung zu kombinieren besteht darin, in einer Zusammensetzung solche Komponenten zu kombinieren, die beide Anforderungen erfüllen können. Insbesondere geeignet sind hierfür Mischungen aus Polyacrylaten mit hoher Wasseraufnahme- und -haltekapazität und Hydrokolloiden wie z. B. natürlichen Polymeren wie Polysaccharide, insbesondere Alginate, Hyaluronsäure, Carrageene oder Cellulosen, mit guten hydratisierenden Eigenschaften.

Weiter ist es insbesondere in der kosmetischen Anwendung aber auch in der äußerlichen therapeutischen Anwendung erwünscht, eine Zusammensetzung bereitzustellen, die gewissermaßen als Leave-on Produkt ausgestaltet ist. Dafür muß das Produkt derart ausgestaltet sein, dass es praktisch vollständig und nahezu rückstandsfrei auf der Haut oder im Haar verbleiben kann. Weiter müssen solche Leave-on Produkte eine gewisse Viskosität aufweisen um dem Anwender eine gehaltvolle und gut zu applizierende Zubereitung zur Verfügung stellen zu können. Das Bestreben geht somit dahin, Zusammensetzungen zur Verfügung zu stellen, die mit wenig Füll- oder Hilfsstoff-Material, idealerweise

jedoch hohen Wirkstoffgehalten dennoch über eine hohe Anwendungsviskosität verfügen.

Für diese Zwecke eignen sich insbesondere Polymere auf Basis von Polyacrylsäuren und deren Salzen, insbesondere sogenannte Superabsorber oder auch Hydrogele.

Wasserhaltige Hydrogele für die Wundversorgung werden häufig auch als amorphe Gele in Tuben oder Spritzen angeboten. Solche gelförmigen Hydrogele sind beispielsweise bekannt aus der EP 583170 auf Basis reiner synthetischer Polymere wie z. B. Polyacrylsäure, oder auf Basis reiner natürlicher Strukturpolymere wie aus der WO 97/03710.

Auch gelförmige Mischungen synthetischer und natürlicher Strukturpolymere werden im Stand der Technik offenbart wie z.B. in der EP 737703, die flüssige oder amorphe polymere Hydrogele aus mit Polysacchariden stabilisierten Polyester-Copolymeren zum Gegenstand hat oder aus der EP 1779836, die Hydrodispersionen auf Basis von Mischungen von Polyacrylaten und Hydroxypropylguar offenbart.

Die DE 102005035879 offenbart ein wässriges Hydrogel für die Wundversorgung, das neben einem synthetischen Acrylsäure-Derivat mit hoher Wasseraufnahmekapazität mindestens ein gelbildendes Polysaccharid sowie ein Elektrolytgemisch aus mindestens zwei unterschiedlichen Elektrolyten aufweist. Dieses Hydrogel weist einen Wassergehalt von mindestens 50 Gew.-% auf und verfügt offenbarungsgemäß über besonders gute absorbierende und gleichzeitig hydratisierende Eigenschaften bei guter Modellierfähigkeit und Gelformstabilität.

Nachteilig an solchen flüssigen, halbflüssigen oder gelförmigen Applikationsformen ist prinzipiell die hohe Anfälligkeit gegenüber Verderb und mikrobiellem Befall, was eine Konservierung notwendig macht. Dabei ist insbesondere der Zusatz von chemischen Konservierungsstoffen eher unerwünscht. Physikalische Konservierungsverfahren wie Bestrahlung oder Hitzebehandlung wirken sich direkt auf die Struktur der Gelbildner und damit auf die Viskosität der Zusammensetzung aus. Diese Verfahren führen häufig zu nur schwer einstellbaren und unkontrollierbaren Viskositätsveränderungen.

Desweiteren weisen Zubereitungen in Gel-, Pulver- oder Granulatform durch ihre unzusammenhängende, ungeformte, forminstabile Erscheinung in der Regel eine schlechte Applizierbarkeit in und auf der Wunde auf. Auch sind solche unzusammenhängenden Zusammensetzungen schlecht in der Menge zu dosieren. Zudem ist die gleichmäßige Verteilung der pulver-/gelförmigen Zubereitungen ein Problem.

Bevorzugt werden daher solche Applikationsformen eingesetzt, die eine feste, zusammenhängende Form aufweisen, die auf die zu applizierenden Behandlungspartien in der Ausgestaltung ihrer Form und Größe gut abgestimmt und angepasst werden können. Zubereitungen, die für Behandlungen der Haut vorgesehen sind, weisen dabei vorzugsweise eine flächige Ausgestaltung auf, die eine einfache und gleichmäßige Applikation ermöglicht. Geeignete Zubereitungen sollten dabei außerdem eine gewisse Formstabilität und einen mechanischen Zusammenhalt besitzen, so dass neben der einfachen ersten Applizierbarkeit idealerweise auch eine weitere Modellierbarkeit auf oder im Behandlungsareal möglich ist. Außerdem ist erwünscht, solche flächigen, trockenen Zubereitungen in an das zu behandelnde Areal angepasster Form und Größe bereitzustellen, wozu ebenfalls eine gewisse mechanische Stabilität der Zubereitungen erforderlich ist.

Um solche Hydrogele oder hydratisierbaren, quellbaren Hydrokolloid-Zusammensetzungen in einer kohärenten, flächigen Form mit den gewünschten Eigenschaften hinsichtlich Konservierung, Applizierbarkeit, Formstabilität sowie Modellierbarkeit bereitstellen zu können, werden diese häufig in getrockneter Form z. B. in Form von Filmen, Sheets, Pads oder Kompressen angeboten. Auch in Hinblick auf die Lagerung und den Transport sind trockene Zubereitungen vorteilhaft gegenüber wasserhaltigen oder feuchten Zubereitungen.

Um Hydrokolloide oder Hydrogel-Komponenten, insbesondere Polyacrylatgele in eine flächige Form z.B. Sheet- oder Auflagenform bringen zu können, ist eine gewisse mechanische Stabilisierung der getrockneten Hydrogel-Zusammensetzung notwendig, da das Material andernfalls nach der Trocknung z.B. bei einer mechanischen Nachbearbeitung wie Schneiden in die gewünschte Form oder auch bereits bei der Handhabung insbesondere großflächiger getrockneter Zusammensetzungen leicht in ein Granulat oder Pulver zerfällt.

Aus dem Stand der Technik bekannt sind solche getrockneten Hydrogel- oder Polymer-Zubereitungen, die durch Aufbringen auf einen festen, vorzugsweise textilen und/oder unlöslichen Träger mechanisch stabilisiert und in eine applizierbare flächige Form gebracht werden.

So beschreibt die WO 01/82886 ein gefriergetrocknetes kosmetisches "patch" aus einer rehydratisierbaren Hydrokolloid-Zusammensetzung, die natürliche Polysaccharide wie Alginate, halbsynthetische Hydrokolloide wie Cellulosen oder auch synthetische Polymere wie Polyacrylate enthalten kann, und das erhältlich ist durch Aufbringen der Hydrokolloid-Zusammensetzung auf einen daran adhärierenden festen Träger z. B. in Form eines Vlieses, eines Netzes oder eines vernetzten Schaumes z. B. aus Polyurethan und anschließender Gefriertrocknung. Der adhärierende Träger ist hierbei maßgeblich für den Erhalt einer ausreichenden mechanischen Stabilität während des Gefriertrocknungsprozesses und bei der späteren Handhabung und Applikation der "patches" und ermöglicht erst die Bereitstellung der Zusammensetzung in der gewünschten "patch"- oder Maskenform. Bei der Verarbeitung von Polyacrylsäure-Polymeren in der kosmetischen Industrie wird generell der pH-Wert neutralisiert, so auch bei der Verarbeitung der beschriebenen Zusammensetzungen, die mittels Natriumhydroxid neutralisiert werden. Dies führt zu der mangelhaften Stabilität derartiger Zusammensetzungen in der Gefriertrocknung und späteren Handhabung, weswegen das Aufbringen auf einen

unlöslichen, stabilisierenden Träger notwendig ist.

Weitere trockene Hydrokolloid- oder Hydrogel-Zusammensetzungen in Form flächiger Sheets oder Masken, die durch Aufbringen der Hydrokolloidzusammensetzung auf einen festen Träger stabilisiert werden, sind bekannt aus der WO 99/20318 und WO 97/41900, worin wasserlösliche Hydrokolloid-Zubereitungen, z. B. auf Basis von Alginaten, durch Gießen auf ein festes Substrat aufgebracht und auf diesem zusätzlich durch Vernetzung mit Metallionen stabilisiert werden, bevor die Zubereitungen einer Gefriertrocknung unterzogen werden.

Auch die in der DE 60113937 (hervorgegangen aus der o.g. WO 01/82886) offenbarten Masken oder Patches aus einer Mischung synthetischer Strukturpolymere wie z.B. Polyacrylat und natürlicher Hydrokolloide wie z.B. Alginat oder Cellulose sind durch Aufbringen auf eine feste faserige Trägermatrix derart stabilisiert, dass die auf dem Träger gefriergetrockneten Zubereitungen in die gewünschte Form zugeschnitten werden können.

Nachteilig an solchen auf einem Träger fixierten Zubereitungen ist die Inhomogenität des Materials sowie insbesondere die Notwendigkeit der Entfernung der unlöslichen, meist synthetischen Trägermatrix von oder aus der zu behandelnden Körperpartie oder auch Wunde. Insbesondere bei Verwendung von zu einem Gel rehydratisierbaren Hydrokolloidzusammensetzungen in der Wundbehandlung sind unlösliche oder nicht-rehydratisierbare Bestandteile in der Zusammensetzung unerwünscht, da diese beispielsweise nicht resorbiert oder nur schwer aus der Wunde mit dem Wundexsudat ausgespült werden können. Auch bei einer kosmetischen Behandlung unter Verwendung einer zu einem viskosen Gel rehydratisierbaren Hydrogel-Zusammensetzung ist eine unlösliche Trägermatrix unerwünscht, da eine solche Trägerschicht die Einarbeitung der in der Zusammensetzung enthaltenen Wirk- und Aktivstoffe insbesondere z. B. durch Massage der hydratisierten Gelzusammensetzung auf der Haut, behindert. Auch ist es schwierig, während der Anwendung die für die Behandlung relevante Hydrogel-Zusammensetzung wieder von der für die tatsächliche Behandlung in der Regel nicht relevanten Trägermatrix zu trennen. Beim Entfernen der Trägermatrix wird dabei immer eine gewisse Menge daran anhaftender oder darin eingebetteter Hydrogel-Zusammensetzung mit entfernt werden, die dann für die eigentliche Behandlung nicht mehr zur Verfügung steht. Dadurch ist zum einen eine reproduzierbare und dosisgenaue Applikation des Behandlungsmittels oder von Wirkstoffen nicht möglich und zum anderen werden hierdurch Materialressourcen ungenutzt vergeudet, was auch aus wirtschaftlichen Gesichtspunkten unerwünscht ist.

Die Bereitstellung von mechanisch stabilen, getrockneten Hydrokolloid-Zusammensetzungen, insbesondere in flächiger Ausgestaltung, die ohne Verwendung solcher unlöslichen, flächigzusammenhängenden Trägermaterialien stabilisiert sind, ist beispielsweise bekannt aus der DE 4328329 oder der WO 01/78692, worin Membranen oder Masken auf Basis von gefriergetrockneten natürlichen Strukturpolymeren, etwa auf Basis von Natrium-Alginaten offenbart sind. Dabei werden die Matrices der DE 4328329 durch den Zusatz von losen Spinnfasern wie z. B. textilen Rayonfasern oder durch eine Calcium-Vernetzung derart stabilisiert, dass die gegossenen und gefriergetrockneten Blöcke in die gewünschte Maskenform zugeschnitten werden können. Die gefriergetrockneten Gelmatrizen nach der WO 01/78692 sind ebenfalls mechanisch stabilisiert durch eine Teilvernetzung, insbesondere durch Calciumionen. Beide Dokumente offenbaren dabei jedoch keine Mischungen von natürlichen Hydrokolloiden mit synthetischen Strukturpolymeren wie insbesondere solchen auf Basis von Polyacrylat und seinen Derivaten.

Auch in den Dokumenten WO 95/19795 oder GB 2401879 werden lediglich auf natürlichen Polymeren basierende trockene sheet- oder trockene, schwammförmige Hydrogel-Zubereitungen offenbart, die eine Stabilisierung durch den Zusatz von Textilfasern z. B. aus Rayon (Viskose) oder Baumwolle oder von nicht-quellenden synthetischen Polymerfasern z. B. auf Basis von Polyamid, Polyester oder Polyether erhalten.

Die WO 03/051412 hingegen offenbart gefriergetrocknete, adsorbierende Schwammmaterialien, beispielsweise in Form uniformer Sheets, die aus synthetischen Polyacrylsäure-Polymeren erhältlich sind, die ebenfalls mit solchen unlöslichen, synthetischen textilen Fasermaterialien wie insbesondere mit Carboxymethylcellulose-Fasern stabilisiert sind.

Der Zusatz unlöslicher Textilfasern oder anderer nichtquellender synthetischer Faserbestandteile zu solchen rehydratisierbaren und unter Gelbildung löslichen Hydrogel-Zubereitungen zur Stabilisierung ist insofern unerwünscht, als dass diese unlöslichen textilen und nicht-quellenden Faserbestandteile in dem rehydratisierten Gel eine unlösliche Komponente bilden, die insbesondere bei einer kosmetischen Behandlung einer aufwändigen und unerwünschten Abreinigung im Anschluss an die eigentliche Behandlung bedarf. Auch sind solche unlöslichen und nicht-quellbaren Bestandteile z. B. in einer Wunde nicht resorbierbar oder abbaubar und als partikuläre Fremdkörper in einer Wunde potentielle Auslöser für Granulationsherde, bedingt durch Abkapselungsvorgänge. Solche Granulationsherde stellen außerdem ein potentielles Infektionsrisiko dar, was insbesondere in an sich schon schlecht heilenden oder chronischen Wunden mit generell sehr instabilen Wundmilieubedingungen unerwünscht ist. Die partikulären Fremdkörper müssen meistens aufwendig durch Spülen aus der Wunde entfernt werden.

Bei einer Stabilisierung durch chemische Vernetzung der Zusammensetzung erfolgt prinzipiell eine Überführung der löslichen Polymerbestandteile in eine wasserunlösliche, unter üblichen Anwendungsbedingungen irreversibel fest verbundene Zusammensetzung, wodurch eine Bereitstellung trockener Hydrokolloid-Zusammensetzungen insbesondere in flächiger Sheet-, Schaum- oder Schwamm-Ausgestaltung möglich wird.

Derart durch Vernetzung stabilisierte Zubereitungen sind neben der bereits zitierten DE 4328329, WO 01/78692,

WO 99/20318 oder WO 97/41900 beispielsweise auch aus der WO 97/39781 und der WO 96/13285 bekannt, worin durch Vernetzung stabilisierte, gefriergetrocknete Schwämme und Schäume mit hoher Absorptionskapazität auf Basis natürlicher Hydrokolloide für die Wundbehandlung offenbart werden. Gefriergetrocknete Zusammensetzungen aus Mischungen von mindestens einem Polymer auf Basis von Polyacrylsäuren und Salzen davon mit mindestens einem natürlichen Polymer wie insbesondere Alginaten, Hyaluronsäure, Carrageen oder Cellulosen, die in Form einer homogenen, mechanisch stabilen, dabei jedoch Träger- bzw. Faserfreien Matrix vorliegen, werden auch hier nicht offenbart.

Solche Mischungen sind hingegen prinzipiell Gegenstand der WO 03/068843, worin sogenannte Interpolyelektrolyt-Komplexe aus ionisch vernetzten Mischungen von Polyacrylsäure-Polymeren mit Chitosanen, die zur Gruppe der natürlichen Polymere gehören, erhalten werden. Die darüber erhältlichen Gele können auch lyophilisiert werden, wobei die Ausgestaltung in Form zusammenhängender Matrices oder Auflagen nicht beschrieben wird. Vielmehr werden die getrockneten Gele zu partikulärem Material vermahlen. Dies lässt auf eine nicht ausreichende Stabilität zur Bereitstellung in Form zusammenhängender Sheets schließen.

Solche Mischungen sind weiterhin auch bekannt aus der DE 19710369, die wasserunlösliche, chemisch vernetzte Vliese oder Masken für die kosmetische Anwendung auf Basis von Chitosan und - derivaten offenbart, die mit Vernetzungsmitteln wie insbesondere Polyacrylsäuren vernetzt sind. Die ausreichende Stabilität zur Bereitstellung in Form von Masken oder Vliesen wird hier durch die chemische Vernetzung der Polyacrylat-Polymers mit dem natürlichen Polymer Chitosan, das zur Gruppe der kationischen Polymere gehört, erreicht, hingegen sind Mischungen aus Polyacrylat/-derivaten mit sogenannten anionischen natürlichen Polymeren wie z. B. Alginaten, Hyaluronsäure, Carrageen und/oder mit Cellulosen auch hier nicht Gegenstand des Offenbarungsgehaltes.

Durch Vernetzungsreaktionen erhaltene Stabilisierungen können nicht oder nur sehr schwer unter Auflösung und Gelbildung rehydratisieren und somit lösliche Zusammensetzungen bilden. Teilvernetzungen zum Erhalt rehydratisierbarer Gelverbindungen, wie in der WO 01/78692 oder in der WO 03/068843 beschrieben, sind insbesondere bei Hydrogelen auf Basis von Mischungen aus Polyacrylat-/derivaten und natürlichen Hydrokolloiden nicht geeignet, um eine ausreichende Stabilisierung zur Bereitstellung mechanisch stabiler, schneidbarer Zusammensetzungen zu erhalten. Zudem erfordert die Auflösung einer solchen chemischen Vernetzung den Zusatz spezieller chemischer Reaktionsmittel in der Hydratisierungsflüssigkeit, was einerseits die Flexibilität in der Wahl der Zusammensetzung solcher Flüssigkeiten stark beeinträchtigt und außerdem aufgrund möglicher Unverträglichkeitspotentiale solcher chemischer Zusätze unerwünscht ist.

Die Aufgabe der vorliegenden Erfindung bestand somit darin, eine gefriergetrocknete Zusammensetzung zur Verfügung zu stellen, die eine hohe Feuchtigkeitsaufnahme- und -haltekapazität aufweist und dabei dennoch auch eine hydratisierende, also befeuchtende Wirkung besitzt und die in Form einer flächigen Ausgestaltung z. B. in Form von Masken, Auflagen, Sheets oder Pads, sowie in Form großformatiger Formkörper bereitstellbar ist. Darüber hinaus sollte diese Zusammensetzung unter Zufuhr von hydrophilen Flüssigkeiten unter Bildung eines homogenen, feindispersen Gels, welches im Wesentlichen frei von makroskopischen Partikeln oder Faserbestandteilen ist, rehydratisierbar sein, um insbesondere für die kosmetische und therapeutische Hautbehandlung geeignet zu sein. Durch die Kombination von natürlichen strukturbildenden Polymeren wie insbesondere solchen aus der Gruppe der anionischen Polymere, insbesondere aus der Gruppe der Polysaccharide wie z. B. Alginaten mit mindestens einem Polymer auf Basis von Polyacrylsäuren und Salzen davon, wie z. B. insbesondere solchen aus der Gruppe der Carbomere können durch Gefriertrocknung schwammartige Zusammensetzungen oder Formkörper hergestellt werden, die über eine ausreichende mechanische Stabilität verfügen, und die durch Zuschneiden in Applikationsformen wie solche in Form von Masken, Sheets, Auflagen oder Pads überführt werden können. Solche erfindungsgemäßen gefriergetrockneten Zusammensetzungen sind unter Gelbildung weitestgehend rückstandsfrei und schnell rehydratisierbar und verfügen über eine hohe Feuchtigkeitsaufnahme- und -haltekapazität sowie eine gute hydratisierende bzw. befeuchtende Wirkung.

Gefriergetrocknete Zusammensetzungen, die synthetische Polyacrylsäure-Polymere in Kombination mit einem natürlichen Polymer aus der Gruppe der Alginate enthalten sind beispielsweise bekannt aus der WO 99/65538 und der WO 01/28600. Darin werden Wundauflagen beschrieben, die neben den o.g. polymeren Hydrokolloiden außerdem iodhaltige Wirkstoffe enthalten. Die Zusammensetzungen der WO 99/65538 werden außerdem bevorzugt bei einem pH-Wert von 3 - 6,5 hergestellt.

Weiterhin sind gefriergetrocknete Zusammensetzungen in Form eines zusammenhängenden, schwammartigen Sheet-Materials enthaltend synthetische Polyacrylsäure-Polymere in Kombination mit einem natürlichen Polymer aus der Gruppe der Hyaluronsäure und ihrer Derivate beispielsweise bekannt aus der WO 94/01468, wobei hierin eine Neutralisierung mit anorganischen Basen und kein Arbeiten bei sauren pH-Werten beschrieben wird.

Es wurde nun überraschend gefunden, dass derartige gefriergetrocknete Zusammensetzungen in Hinblick auf ihre Flüssigkeitsaufnahme- bzw. Flüssigkeitshaltekapazität entscheidend verbessert werden können, wenn solche synthetischen Polymere aus der Gruppe der Polyacrylsäuren mit natürlichen Polymeren aus der Gruppe der Alginate sowie mit einem weiteren natürlichen Polymer aus der Gruppe der Hyaluronsäure und ihrer Derivate sowie ggf. aus der Gruppe der Carrageene kombiniert werden.

Weiterhin wurde überraschend gefunden, dass mit den erfindungsgemäßen Zusammensetzungen die Flüssigkeitshaltekapazität insbesondere für physiologische Flüssigkeiten entscheidend verbessert bzw. stabil gehalten werden konnte. Dies ist vor allem im Bereich der Wundbehandlung, z.B. beim Wundexsudatmanagement oder für die Applikation von physiologischen Wirkstofflösungen, aber auch in der Kosmetik bei Verwendung von physiologischen, und damit besonders verträglichen, Kosmetik- oder Aktivatorlösungen von besonderer Bedeutung.

Dabei bleiben die o.g. gewünschten Eigenschaften wie gute Verarbeitbarkeit, mechanische Stabilität, insbesondere hohe Schneidstabilität, gute Handhabbarkeit und hohe Verträglichkeit etc. erhalten. Eine derartige Verbesserung ist damit insbesondere für die genannten Anwendungsgebiete in der kosmetischen und medizinischen Verwendung von großem Interesse.

Dabei können solchen Zusammensetzungen außerdem weitere natürliche oder synthetische Polymere wie eben Carrageen oder beispielsweise Collagen oder Cellulose oder -derivate sowie auch Wirk- und/oder Hilfsstoffe zugesetzt werden.

Keines der genannten Dokumente offenbart eine gefriergetrocknete Zusammensetzungen in Form eines mechanisch stabilen, zusammenhängenden, schwammartigen Sheet-Materials enthaltend synthetische Polyacrylsäure-Polymere in Kombination mit einem natürlichen Polymer aus der Gruppe der Alginate und einem weiteren natürlichen Polymer aus der Gruppe der Hyaluronsäure und ihrer Derivate sowie ggf. aus der Gruppe der Carrageene und/oder aus der Gruppe der Collagene und/oder Cellulosen. Weiterhin offenbart keines der Dokumente derartige Zusammensetzungen mit einem sauren pH-Wert von 3 bis 6,5 bzw. entsprechende Zusammensetzungen die durch Arbeiten bei derartigen sauren pH-Werten erhältlich sind.

Bei der Kombination von Polyacrylaten und -derivaten mit natürlichen Polymeren oder natürlichen Hydrokolloiden, wie solchen aus der Gruppe der Polysaccharide, insbesondere Alginate, Carragenan oder Cellulosen, ist die sehr spezielle Interaktion zwischen den Polymerkomponenten in der wässrigen Mischung zu berücksichtigen.

Bei der Substanzklasse der Carbomere (synthetische Acrylsäurepolymere) handelt es sich um raumgreifende verzweigte Polymere, die als wesentliche funktionelle Einheiten Carbonsäuregruppen tragen. Diese Carbonsäuren können, als Funktion des pH-Wertes, unterschiedliche Ladungszustände aufweisen.

Wässrige Carbomer-Lösungen reagieren sauer, mit zunehmendem pH-Wert, bzw. mit zunehmender Neutralisation und Verschiebung des pH-Werts in den alkalischen Bereich, erfahren die Säuregruppen eine Deprotonierung, wodurch das Polymergerüst negativ geladen wird. Durch die Ladungsverschiebung erfolgt eine intramolekulare Abstoßung der negativ geladenen Polymerkette, welches in einer räumlichen Entfaltung resultiert. Die Carboxylatgruppen werden hydratisiert, das Polymermolekül nimmt Wasser auf, es quillt. Als messbaren Effekt der Carbomer Neutralisation ergibt sich eine Zunahme der intrinsischen Viskosität.

Diese durch intramolekulare elektrostatische Abstoßung bedingte Molekülentfaltung und die damit einhergehende Viskositätserhöhung einer wässrigen Carbomerlösung, ist stark pH-Wert abhängig. Dieser Vorgang ist reversibel, d.h. mit zunehmender Protonierung neutralisiert man das Polymergerüst, die elektrostatische Abstoßung verringert sich und das Molekül fällt sozusagen zusammen, wodurch auch die Lösungsviskosität abnimmt.

Die Reduzierung der intrinsischen Viskosität kann auch durch den Einfluss von Salzen oder Elektrolyten, wie sie beispielsweise in physiologischen Flüssigkeiten enthalten sind, erzielt werden. Die Viskosität Carbomer-haltiger Lösungen stellt also ein reversibles System mit hoher pH-Wert und Elektrolyt-Sensitivität dar.

Ein durch Neutralisation entfaltetes Carbomermolekül ist in der Lage, in einer Zusammensetzung mit anderen Polymeren Wechselwirkungen einzugehen. Insbesondere elektrostatische Wechselwirkungen unterschiedlich geladener Komponenten können hier zu einem großen Wechselwirkungspotential und damit zu einer guten mechanischen Stabilisierung anschließend getrockneter Matrizen führen. Dieses Prinzip machen sich insbesondere sogenannte "Interpolyelectrolyte Complexes" wie in "Matrix Polymeric Excipients: Comparing a Novel Interpolyelectrolyte Complex with Hydroxypropylmethylcellulose; Zhilei Lu et al., Drug Delivery, Vol. 15, Issue 2, 2008, 87-96" beschrieben, zunutze. Dabei werden unter dem Begriff "Interpolyelectrolyte Complexes" Mischungen unterschiedlich geladener Polymere verstanden, hier insbesondere eine Mischung aus kationischem Chitosan (Polyaminosaccharid, welches über freie Aminogruppen verfügt und damit in saurer Lösung als Polykation wirken kann) und anionischem Carbomer wie auch schon in der zitierten DE 19710369 und WO 03/068843 dargestellt. Durch die elektrostatischen Kräfte zwischen den entgegengesetzt geladenen Polymer-Komponenten, kommt es zur Ausbildung ionischer Wechselwirkungen, welche deutlich stärker sind als z. B. van der Waals-Kräfte oder Wasserstoffbrückenbindungen. Die Polymere erfahren quasi eine Stabilisierung durch "ionische" Assoziation bzw. Vernetzung.

Setzt man hingegen Mischungen aus gleichsinnig geladenen Polymeren, wie beispielsweise Mischungen aus einem anionischen Carbomer und einem anionischen Hydrokolloid, wie beispielsweise Alginat oder Hyaluronsäure bzw. Carrageen bzw. Collagen, ein, so fehlt in dieser Zusammensetzung die stabilisierende ionische Komponente. In dem gewählten Fall Carbomer/Alginat beispielsweise erhält man bei einer zu starken Neutralisation sogar einen durch die elektrostatische Abstoßung bedingten destabilisierenden Beitrag. Eine im trockenen Zustand stabile Zubereitung, enthaltend solche gleichsinnig geladenen Hydrokolloid-Mischungen kann nur erhalten werden, wenn die destabilisierenden Kräfte zwischen den unterschiedlichen Polymerkomponenten möglichst klein gehalten werden, so daß die trockene Zusammensetzung allein über van der Waals-Kräfte oder über Wasserstoffbrückenbindungen erfolgt, wobei solche

Wechselwirkungen deutlich geringer stabilisierend wirken, als die elektrostatischen Wechselwirkungen gegensinnig geladener Polymere.

Kombiniert man in einer hochviskosen Lösung, enthaltend maximal entfaltete Carbomere, die im entfalteten, gequollenen Zustand eine hohe negative Ladungsdichte aufweisen, mit weiteren anionischen Hydrokolloiden bzw. negativ geladenen Polymeren wie z.B. Alginatlösungen, so weisen die gefriergetrockneten Endprodukte solcher Lösungen aufgrund der elektrostatischen Abstoßung der gleichsinnig geladenen Polymerkomponenten sowie aufgrund des Einflusses weiterer Zusammensetzungsbestandteile häufig eine unbefriedigende mechanische Stabilität auf. Verringert man z. B. durch pH-Wert-Verschiebung die Ladungsdichte der anionischen Polymere in der Lösung, um die elektrostatische Abstoßung der Polymere zu vermindern, so erfährt das Carbomer mit der damit einhergehenden Protonierung der Carboxylatgruppen einen Abfall der intrinsischen Viskosität.

Somit weisen die gefriergetrockneten Endprodukte solcher Zusammensetzungen aus Carbomer und gleichsinnig geladenen Hydrokolloiden meist entweder eine ungenügende mechanische Stabilität aufgrund einer ungenügenden intrinsischen Viskosität des Carbomers oder eine ungenügende mechanische Stabilität aufgrund der elektrostatischen Abstoßung der gleichsinnig geladenen Polymere auf. Dieser Effekt wird zusätzlich verstärkt, wenn weitere elektrolytisch wirkende Substanzen in solchen Zusammensetzungen enthalten sind, auf die die Carbomere wie oben ausgeführt sensitiv reagieren.

Aus den vorstehenden Gründen ist somit bevorzugt, die gefriergetrockneten erfindungsgemäßen Zusammensetzungen unter Einstellung des pH-Werts auf einen Wert zwischen pH 3,0 und 6,5, bevorzugt zwischen pH 4,0 und 6,0, bevorzugter zwischen pH 4,5 und 5,5, besonders bevorzugt pH 5,0 herzustellen, um dadurch besonders mechanisch stabile, gefriergetrocknete Zusammensetzungen aus hochviskosen Mischungen eines Carbomers mit einem natürlichen Hydrokolloid, insbesondere mit einem anionischen Hydrokolloid, wie z.B. einem Alginat und mit verbesserten Flüssigkeitsaufnahme- und - halteeigenschaften, insbesondere in Hinblick auf physiologische Flüssigkeiten, zu erhalten. Durch eine solche pH-Wert Einstellung soll ein unerwünschter Viskositätsabfall oder eine nachteilige zusätzliche Stabilisierung der Zusammensetzung z. B. durch Zugabe unlöslicher Bestandteile wie Trägermatrices oder Fasern oder durch chemische Vernetzung vermieden werden..

Bevorzugt sind somit solche gefriergetrockneten Zusammensetzungen, deren 1 gewichtsprozentige Lösung oder Suspension in Wasser bei 20 °C einen pH-Wert von 3,0 bis 6,5, bevorzugt zwischen pH 4,0 und 6,0, bevorzugter zwischen pH 4,5 und 5,5, besonders bevorzugt pH 5,0 aufweist.

Darüber hinaus zeigte sich überraschend, dass solche erfindungsgemäßen gefriergetrockneten Zusammensetzungen mit einem pH-Wert von pH 3,0 und 6,5, bevorzugt zwischen pH 4,0 und 6,0, bevorzugter zwischen pH 4,5 und 5,5, besonders bevorzugt pH 5,0 außerdem eine optimale Benetzungsgeschwindigkeit aufweisen, was für die Rehydratisierung und Überführung in ein Gel für die kosmetische Anwendung oder den Einsatz als feuchtigkeitsabsorbierende Wundauflage von essentieller Bedeutung ist.

Weiter hat sich überraschend gezeigt, dass die erfindungsgemäßen gefriergetrockneten Zusammensetzungen mit einem pH-Wert von pH 3,0 und 6,5, bevorzugt zwischen pH 4,0 und 6,0, bevorzugter zwischen pH 4,5 und 5,5, besonders bevorzugt pH 5,0 besonders hohe optische Dichten aufweisen. Dabei zeigt sich ein direkter Einfluss des eingestellten pH-Wertes auf die zu erzielende optische Dichte der gefriergetrockneten Zusammensetzungen.

Die Ladungsdichte des Carbomers in diesem pH-Wertebereich ist hoch genug um eine ausreichende intramolekulare Abstoßung der Carboxylatgruppen zu gewährleisten (hohe intrinsische Viskosität, ausreichend hoher hydrodynamischer Radius), ohne die intermolekulare Abstoßung des negativ geladenen Carbomers mit den weiteren anionischen natürlichen Polymeren oder weiterer elektrolytisch wirksamer Bestandteile zu groß werden zu lassen. In diesem pH-Wertebereich ist die Stabilisierung der gefriergetrockneten Matrix über Wasserstoffbrücken- und van-der-Waals-Wechselwirkungen ausreichend hoch, dass mechanisch stabile, insbesondere schneidstabile, optisch dichte Matrizen erhalten werden. Erhöht man den pH-Wert auf > pH 6,5, so kann die elektrostatische Abstoßung der gleichsinnig geladenen Polymerbestandteile und die negative Auswirkung auf die stabilisierenden Wasserstoffbrückenbindungen und van der Waals-Kräfte so hoch werden, dass die gefriergetrockneten Zusammensetzungen mechanisch zu instabil werden, um stabile großformatige Formkörper zu bilden oder um durch Schneiden in die gewünschten Formen überführt werden zu können. Auch die Benetzbarkeit solcher Zusammensetzungen verschlechtert sich drastisch, wodurch ein pH-Wert der gefriergetrockneten Zusammensetzung von maximal pH 5,5 besonders bevorzugt ist.

Aus dem Stand der Technik sind keine gefriergetrockneten Zusammensetzungen mit einer derartigen Polymer-Zusammensetzung bekannt, die ohne zusätzliche Stabilisierungsbestandteile herstellbar sind und über entsprechende physikalische, mechanische und chemische Eigenschaften verfügen.

Die Erfindung stellt somit gefriergetrocknete Zusammensetzungen bereit, die mindestens ein Polymer auf Basis von Polyacrylsäuren und Salzen davon sowie mindestens ein natürliches Polymer, sowie gegebenenfalls mindestens ein weiteres von diesen vorgenannten Polymeren verschiedenes natürliches oder synthetisches Polymer, sowie gegebenenfalls einen oder mehrere Wirk- und/oder Hilfsstoffe enthalten. Weiterhin betrifft die Erfindung ein Verfahren zur Herstellung solcher gefriergetrockneten Zusammensetzungen, die Kombination solcher gefriergetrockneten Zusammensetzungen in Kit-of-parts Anordnungen zusammen mit wässrigen Lösungen sowie die Verwendung der gefriergetrockneten Zusammensetzungen und der Kit-of-parts Kombinationen zur kosmetischen und pharmazeutischen Anwendung, insbesondere zur Verwendung als kosmetische Maske sowie als Wundauflage und zur Herstellung von pharmazeutischen Mitteln zur Behandlung von dermalen Wunden wie insbesondere chronischen Wunden, Ulcus cruris oder Dekubitus.

Unter der gefriergetrockneten Zusammensetzung im Sinne der Erfindung versteht man auch eine gefriergetrocknete Hydrogel-Zusammensetzung, insbesondere ein hydrophiles Polymer bzw. eine hydrophile Polymerzusammensetzung, wobei insbesondere hydrokolloide, strukturbildende Polymere eine solche Hydrogel-Zusammensetzung bilden, und die in der Lage sind, in wässrigen Flüssigkeiten unter Volumenzunahme und Viskositätserhöhung zu quellen. Dadurch weisen solche Zusammensetzungen im Sinne der vorliegenden Erfindung eine hohe Flüssigkeitsaufnahme- und - speicherkapazität bzw. im gequollenen, hydratisierten Zustand einen hohen Wassergehalt auf.

Die gefriergetrockneten Zusammensetzungen der vorliegenden Erfindung enthalten mindestens ein Polymer auf Basis von Polyacrylsäuren und Salzen davon, speziell Polyacrylsäure, wie Polyacrylate und ihre Derivate, wie solche mit der Grundstruktur

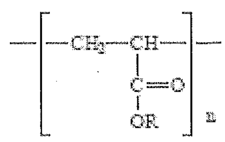

mit R = -H (Polyacrylsäure), bzw. R = Alkylgruppe (Polyacrylate). Diese können amorph und verzweigt, sowie mit weiteren funktionellen Gruppen substituiert oder auch durch Derivatisierung des Grundgerüsts variiert vorliegen. Selektive chemische Veränderungen betreffen dabei insbesondere die Carbonsäuregruppen, z.B. durch Veresterung. Auch ist die Bildung gemischter Co-Polymere (z.B. Block-Copolymere) aus den Polyacrylsäure-Grundgerüsten möglich.

Bei den erfindungsgemäß besonders bevorzugten Acrylsäurepolymeren handelt es sich beispielsweise um Acrylat-Alkylacrylat-Copolymere, insbesondere um solche auf Basis von synthetischen Acrylsäurepolymeren, die auch unter der generischen Bezeichnung "Carbomere" bekannt sind und gemäß USP-NF, British Pharmacopoeia, United States Adopted Names Council (USAN) sowie Cosmetic Toiletries and Fragrance Association (CTFA) die Gruppe der Carbopole (Carbopol®, B. F. Goodrich Company) umfassen.

Bei solchen Carbomeren handelt es sich chemisch um Acrylsäurepolymere, genauer um ein Copolymer aus Acrylsäure und Natriumacrylat, wobei das Verhältnis der Monomere zueinander variieren kann. Auch Homopolymere von Acrylsäure, die mit Pentaerythritallyether, Sucroseallylether oder Propylenallylether vernetzt sind, wie beispielsweise Copolymere aus $C_{10}$-$C_{30}$-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit fallen unter die Gruppe der Carbomere.

Aufgrund der besonders guten Wasseraufnahmefähigkeit der Acrylsäurepolymere, wie insbesondere der Carbomere, sind diese auch unter dem Begriff "Superabsorber" bekannt, womit üblicherweise solche Polymere bezeichnet werden, die in der Lage sind, ein Vielfaches ihres Eigengewichts - bis zum 1000-fachen - an Flüssigkeiten (meist Wasser bzw. destilliertes Wasser) aufzunehmen. Die tatsächliche Aufnahmekapazität hängt dabei insbesondere von der Zusammensetzung der wässrigen Flüssigkeiten ab. Handelt es sich beispielsweise um reines Wasser, so ist die Absorptionskapazität hierfür deutlich höher als beispielsweise für salz- oder elektrolythaltige Flüssigkeiten wie z.B. physiologische Kochsalzlösungen oder physiologische Körperflüssigkeiten. Dies ist möglicherweise auf die bereits diskutierte Elektrolyt-Empfindlichkeit dieser Substanzgruppe zurückzuführen. Wird Elektrolyt-bedingt die Molekülstruktur derart beeinflusst, dass die intrinsische Viskosität abnimmt, das Molekül also seine räumliche Ausdehnung verringert, so ergibt sich eine geringere Kapazität, Wassermoleküle in die Gel- oder Polymerstruktur einzulagern, wodurch im allgemeinen die Wasser-Absorptionskapazität herabgesetzt ist.

Bezüglich der Struktur und Wirkung sowie der damit verbundenen besonderen Eigenschaften hinsichtlich der physikalischen Beschaffenheit von Lösungen sowie der pH-Wert und Elektrolyt-Sensitivität solcher Carbomer-Moleküle wird auf die obigen Ausführungen verwiesen.

Die erfindungsgemäßen gefriergetrockneten Zusammensetzungen enthalten außerdem mindestens ein natürliches Polymer. Dabei wird bevorzugt eines aus der Gruppe der natürlichen strukturbildenden Polymere, bevorzugt aus der Gruppe der Polysaccharide oder Glucosaminoglykane ausgewählt. Dabei wurde überraschend gefunden, dass es mit der erfindungsgemäß vorliegenden Zusammensetzung und Herstellmethode insbesondere möglich ist, anionische hydrokolloid-bildende natürliche Polymere mit den Polyacrylsäuren zu kombinieren. Bevorzugt werden die natürlichen Polymere aus den Polysacchariden ausgewählt. Polysaccharide schließen beispielsweise Homoglykane oder Heteroglykane ein, wie zum Beispiel Alginate, besonders Natriumalginat, Carrageen (hierin ggf. auch unter der gleichbedeutenden englischen Bezeichnung "Carragenan" geführt), Pektine, Pullulan, Tragant, Guar-Gummi, Johannisbrotkernmehl, Agar-Agar, Gummi-Arabikum, Xanthan, Pullulan, natürliche und modifizierte Stärken, Dextrane, Dextrin, Maltodextrine,

Chitosan, Glucane, wie ß-1,3-Glucan, ß-1,4-Glucan, wie Cellulose, Mucopolysaccharide, wie Hyaluronsäure etc. Anionische hydrokolloid-bildende, natürliche Polymere bezeichnet dabei solche in der Natur vorkommenden Polymere, die in wässriger Lösung viskose Lösungen bilden. Sie sind Polyelektrolyte und besitzen ionisierbare funktionelle Gruppen, welche im biologischen (neutralem) pH-Wertebereich eine negative Ladung tragen.

Solche anionischen natürlichen Hydrokolloide sind beispielsweise Alginate, Hyaluronsäure, Carrageen, Carboxymethylcellulose, Gummi arabicum, Agar-Agar, Collagen, Gelatine Typ B, Karaya-Gummi, Pektin, Tragant, Polymethylvinylethermaleinanhydrid (Gantrez) etc.

Die erfindungsgemäß bevorzugt eingesetzten natürlichen Polymere, wie insbesondere die aus der Gruppe der Polysaccharide weisen zweckmäßig durchschnittliche Molmassen von etwa $10^3$ bis zu etwa $10^8$, bevorzugt etwa $10^4$ bis $10^6$ auf.

Erfindungsgemäß besonders bevorzugt sind aus der Gruppe der natürlichen Polymere solche aus der Gruppe der Alginate, wie insbesondere Natrium-Alginat. Bevorzugt sind insbesondere calciumfreie Natrium-Alginate, (Natrium-Alginat mit einem Calciumgehalt < 3 Gew.-%, bevorzugter < 2 Gew.-%, noch bevorzugter < 1,5 Gew.-%).

Weiterhin können in der Zusammensetzung auch ein oder mehrere weitere Polymere aus der Gruppe der natürlichen Polymere enthalten sein, wie z. B. solche wie vorstehend aufgeführt oder auch solche wie beispielsweise Collagene oder deren Derivate wie z.B. lösliches oder unlösliches tierisches oder pflanzliches Collagen, insbesondere z. B. Fischcollagen. Es ist jedoch auch möglich, ein weiteres von den vorstehend genannten unterschiedliches Polymer, z.B. solche aus der Gruppe weiterer synthetischer und/oder modifizierter natürlicher Polymere auszuwählen. Solche synthetischen oder modifizierten natürlichen Polymere schließen beispielsweise ein: Celluloseether, Polyvinylalkohol, Polyvinylpyrrolidon, synthetische Cellulosederivate, wie Methylcellulose, Carboxycellulose, Carboxymethylcellulose, Celluloseester, Cellulosesether wie Hydroxypropylcellulose, Polyacrylsäure, Polymethacrylsäure, Poly(methylmethacrylat) (PMMA), Polymethacrylat (PMA), Polyethylenglykole etc.

Es ist bevorzugt, dass mindestens ein weiteres natürliches Polymer ausgewählt ist aus der Gruppe der Carageene, wie insbesondere Carrageenan und/oder aus der Gruppe der Mucopolysaccharide wie insbesondere Hyaluronsäure und/oder aus der Gruppe der Collagene, wie insbesondere Fischcollagen.

Weiter ist bevorzugt, dass mindestens ein weiteres Polymer aus der Gruppe der synthetischen und/oder modifizierten natürlichen Polymere aus der Gruppe der modifizierten Cellulosen ausgewählt ist, insbesondere ist hieraus bevorzugt Carboxymethylcellulose, insbesondere Natrium-Carboxymethylcellulose.

Es können auch Mischungen mehrerer Polymere aus der Gruppe der natürlichen Polymere und/oder aus der Gruppe der synthetischen Polymere verwendet werden. Besonders bevorzugt werden Mischungen aus Alginaten und Hyaluronsäure sowie ggf. Carrageen und/oder Collagen und/oder Cellulosen.

Die Polymere der erfindungsgemäßen gefriergetrockneten Zusammensetzung weisen eine gute Biokompatibilität auf und sind insbesondere haut- und schleimhautverträglich und weisen weder bei der Anwendung auf intakter Haut noch beim Einbringen in eine der unteren Hautschichten z.B. in Wunden, die eine Verletzung oder Zerstörung des natürlichen Hautaufbaus aufweisen, ein toxikologisches Potential auf. Auch rufen die erfindungsgemäßen Polymere bei der Applikation keinerlei Irritationswirkungen oder andere Unverträglichkeitsreaktionen hervor. Sie sind pharmakologisch völlig unbedenklich und somit optimal geeignet als Polymermaterial für die erfindungsgemäßen kosmetischen und pharmazeutischen dermalen Verwendungen.

Die erfindungsgemäßen Zusammensetzungen können darüber hinaus auch mindestens einen oder mehrere Wirkstoffe enthalten.

Bevorzugt ist in solchen erfindungsgemäßen gefriergetrockneten Zusammensetzungen mindestens einen Wirkstoff enthalten. Wirkstoffe schließen insbesondere kosmetische oder therapeutische bzw. pharmazeutische, für die äußere Anwendung geeignete Wirkstoffe ein. Dementsprechend handelt es sich bei solchen erfindungsgemäßen gefriergetrockneten Zusammensetzungen bevorzugt um kosmetische oder therapeutische Mittel.

Kosmetische Mittel bzw. unter Verwendung kosmetischer Wirkstoffe hergestellte Mittel im Sinne der Erfindung sind im wesentlichen Mittel im Sinne des Lebensmittel-, Bedarfsgegenstände- und Futtermittelgesetzbuches (L.FGB), d.h., Stoffe oder Zubereitungen aus Stoffen, die dazu bestimmt sind, äußerlich am Menschen zur Reinigung, Pflege, oder zur Beeinflussung des Aussehens oder des Körpergeruchs, oder zur Vermittlung von Geruchseindrücken angewendet zu werden, es sei denn, dass sie überwiegend dazu bestimmt sind, Krankheiten, Leiden, Körperschäden oder krankhafte Beschwerden zu lindern oder zu beseitigen. In diesem Sinne handelt es sich bei den erfindungsgemäß verwendeten kosmetischen Formkörpern beispielweise um Badepräparate, Hautwasch- und - reinigungsmittel, Hautpflegemittel, insbesondere Gesichtshautpflegemittel, Augenkosmetika, Lippenpflegemittel, Nagelpflegemittel, Fußpflegemittel, Haarpflegemittel, insbesondere Haarwaschmittel, Haarkonditionierungsmittel, Haarweichspüler etc., Lichtschutzmittel, Hautbräunungs- und -aufhellungsmittel, Depigmentierungsmittel, Deodorants, Antihydrotika, Haarentfernungsmittel, Insektenrepellents etc. oder derartige Mittel in Kombination.

Beispiele kosmetisch gegebenenfalls auch z.B. dermatologischer, therapeutisch wirksamer Verbindungen schließen ein: Antiaknemittel, antimikrobielle Mittel, Antitranspirationsmittel, adstringierende Mittel, desodorierende Mittel, Enthaarungsmittel, Konditionierungsmittel für die Haut, hautglättende Mittel, Mittel zur Steigerung der Hauthydratation

wie z. B. Glycerin oder Harnstoff, Sonnenschutzmittel, Keratolytika, Radikalfänger für freie Radikale, Antiseborrhöika, Antischuppenmittel, antiseptische Wirkstoffe, Wirkstoffe zur Behandlung der Anzeichen der Hautalterung und/oder Mittel, die die Differenzierung und/oder Proliferation und/oder Pigmentierung der Haut modulieren, Vitamine wie Vitamin C (Ascorbinsäure) und ihre Derivate, wie beispielsweise Glycoside wie Ascorbylglucosid, oder Ester der Ascorbinsäure wie Natrium- oder Magnesium-Ascorbylphosphat oder Ascorbylpalmitat und -stearat L-Ascorbinsäurephosphatester, Alkalimetallsalze wie Natrium- und Kaliumsalze von L-Ascorbinsäurephosphatestern; Erdalkalimetallsalze wie Magnesium- und Calciumsalze von L-Ascorbinsäurephosphatestern; trivalente Metallsalze wie Aluminiumsalze von L-Ascorbinsäurephosphatestern; Alkalimetallsalze von L-Ascorbinsäuresulfatestern wie Natrium- und Kaliumsalze von L-Ascorbinsäuresulfatestern; Erdalkalimetallsalze wie Magnesium- und Calciumsalze von L-Ascorbinsäuresulfatestern; trivalente Metallsalze wie Aluminiumsalze von L-Ascorbinsäuresulfatestern; Alkalimetallsalze wie Natrium- und Kaliumsalze von L-Ascorbinsäureestern; Erdalkalimetallsalze wie Magnesium- und Calciumsalze von L-Ascorbinsäureestern; und trivalente Metallsalze wie Aluminiumsalze von L-Ascorbinsäureestern.

Wirkstoffe mit reizender Nebenwirkung, wie alpha-Hydroxysäuren, ß-Hydroxysäuren, alpha-Ketosäuren, ß-Ketosäuren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, mehrfach ungesättigte Fettsäuren, essentielle Fettsäuren (z.B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, oder dekorative Stoffe wie Pigmente oder Farbstoffe und -partikel für Foundations, Make-up Formulierungen, und andere Mittel zur kosmetischen Verschönerung und farblichen Gestaltung von Augen-, Lippen-, Gesicht etc. sowie abrasive Mittel.

Weiterhin können Pflanzenwirkstoffextrakte bzw. daraus gewonnene Auszüge oder Einzelstoffe erwähnt werden. Allgemein wird der Pflanzenwirkstoffextrakt in der Regel ausgewählt aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskautöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl); Immunstimulantien: Echinacea purpurea (z.B.alkoholische Auszüge, Frischpflanzensaft, Preßsaft), Eleutherokokkus senticosus; Alkaloide: Rauwolfia (z.B. Prajmalin), Immergrün (z.B.Vincamin); weitere Phytopharmaka: Aloe, Roßkastanie (z.B. Aescin), Knoblauch (z.B. Knoblauchöl), Ananas (z.B. Bromelaine), Ginseng (z.B. Ginsenoside), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B.Urtinktur), Mistel (z.B. wässrig-ethanol. Auszug), Phytosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordica charantia, und Aloe Vera Extrakte.

Bevorzugte kosmetische Wirkstoffe sind solche, die eine hohe Instabilität gegen Abbau oder Zersetzung, insbesondere bedingt durch Feuchtigkeitszufuhr, aufweisen und die durch Anwendung des Gefriertrocknungsverfahrens in diesen Zubereitungen in einer gegen Feuchtigkeit stabilisierten Form bereitgestellt werden können.

Ein besonders bevorzugter insbesondere in der Kosmetik verbreiteter Wirkstoff aus der Gruppe dieser instabilen Wirkstoffe ist die Ascorbinsäure (Vitamin C) und ihre Derivate, wie beispielsweise Ascorbylglucosid, L-Ascorbinsäurephosphatester, Alkalimetallsalze wie Natrium- und Kaliumsalze von L-Ascorbinsäurephosphatestern; Erdalkalimetallsalze wie Magnesium- und Calciumsalze von L-Ascorbinsäurephosphatestern; trivalente Metallsalze wie Aluminiumsalze von L-Ascorbinsäurephosphatestern; Alkalimetallsalze von L-Ascorbinsäuresulfatestern wie Natrium- und Kaliumsalze von L-Ascorbinsäuresulfatestern; Erdalkalimetallsalze wie Magnesium- und Calciumsalze von L-Ascorbinsäuresulfatestern; trivalente Metallsalze wie Aluminiumsalze von L-Ascorbinsäuresulfatestern; Alkalimetallsalze wie Natrium- und Kaliumsalze von L-Ascorbinsäureestern; Erdalkalimetallsalze wie Magnesium- und Calciumsalze von L-Ascorbinsäureestern; und trivalente Metallsalze wie Aluminiumsalze von L-Ascorbinsäureestern.

Im Unterschied zu den vorstehend beschriebenen im wesentlichen in der Kosmetik verwendeten Zusammensetzungen handelt es sich bei den therapeutischen Zusammensetzungen (Arzneimittel) um solche, die mindestens einen pharmazeutischen bzw. therapeutischen insbesondere auch dermatologischen Wirkstoff enthalten und die im Sinne des Arzneimittelgesetzes unter anderem dazu bestimmt sind, Krankheiten, Leiden, Körperschäden oder krankhafte Beschwerden zu heilen, zu lindern oder zu verhüten. Erfindungsgemäß sind insbesondere solche Mittel bzw. Wirkstoffe geeignet, die für die äußere oder transdermale Anwendung, insbesondere im Bereich der Wundbehandlung und -heilung

bestimmt sind.

Bei Wirkstoffen für die eine solche dermale oder transdermale Anwendung handelt es sich insbesondere um hautaktive aber auch um transdermale Wirkstoffe. Sie schließen beispielsweise ein: Mittel zur Behandlung von Hautkrankheiten, äußerlich anwendbare Analgetika, z. B. Dextropropoxyphen, Pentazocin, Pethidin, Buprenorphin; Antirheumatika/Antiphlogistika (NSAR), z. B. Indometacin, Diclofenac, Naproxen, Ketoprofen, Ibuprofen, Flurbiprofen, Salicylsäure und - derivate wie Acetylsalicylsäure, Oxicame; Steroidhormone, z. B. Betamethason, Dexamethason, Methylprednisolon, Ethinylestradiol, Medroergotamin, Dihydroergotoxin; Gichtmittel, z. B. Benzbromaron, Allopurinol; Dermatika Externa, Antihistaminika, Antibiotika einschließlich antibakterielle Mittel wie z.B. kolloidales Silber und Silbersalze, Antimykotika, Peptidarzneistoffe, antivirale Wirkstoffe, entzündungshemmende Wirkstoffe, juckreizstillende Wirkstoffe, anästhesierende Wirkstoffe, z. B. Benzocain, Corticoide, Aknemittel, antiparasitäre Wirkstoffe; äußerlich anwendbare Hormone; Venentherapeutika; Immunsuppresiva etc. alle für die dermale oder transdermale Anwendung.

Bevorzugte therapeutische Mittel für die dermale und transdermale Anwendung sind Mittel zur Behandlung von Hautkrankheiten, wie der Neurodermitis, der atopischen Dermatitis etc., und Anti-Herpesmittel, sowie insbesondere solche, die im Bereich der Wundbehandlung, insbesondere zur Behandlung von chronischen Wunden, Dekubitus, Ulcus cruris etc. eingesetzt werden wie beispielsweise Analgetika, z.B. Immunsuppressiva, Hormone, anästhesierende Wirkstoffe, antiparasitäre, fungizide bzw. antimykotische und antibakterielle Wirkstoffe wie insbesondere Silber-haltige Wirkstoffe wie z. B. Silbernitrat, Silberchlorid, Silberjodid oder weitere aus dem Stand der Technik bekannte silber-haltige Wundbehandlungsstoffe, Wirkstoffe zur Unterstützung und Regulierung des Wundmilieus wie insbesondere Elektrolyte, Kieselerde, Mineralstoffe und Spurenelemente wie z.B. Kalium, Magnesium, Calcium, Selen, Iod etc. sowie Wirkstoffe zur Erzielung eines Wunddebridements wie z.B. Collagenasen oder andere, im Stand der Technik bekannte, geeignete proteolytischen Enzyme.

Desweiteren ist denkbar, weitere Wirkstoffe wie Bronchialtherapeutika wie Antiasthmatika, Antitussiva, Mucolytica etc., Antidiabetica wie z.B. Glibenclamid, Hormone, Steroidhormone wie Dexamethason, Herzglycoside wie Digitoxin, Herz- und Kreislauftherapeutika wie z. B. Beta-Blocker, Antiarrhythmika, Antihypertonika, Calcium-Antagonisten etc., Psychopharmaka und Antidepressiva wie z.B. trizyklische Antidepressiva (NSMRI), Serotonin-Wiederaufnahme-Hemmer (SSRI), Noradrenalin-Wiederaufnahme-Hemmer (NRI), Serotonin-Noradrenalin-Wiederaufnahme-Hemmer (SNRI), Monoaminooxidase-Hemmer (MAO-Hemmer) etc., Neuroleptika, Antikonvulsiva oder Antiepileptika, Hypnotika, Sedativa, Anästhetika, Magen-, Darmtherapeutika, Lipidsenker, Analgetika wie z.B. Antimigränemittel, Paracetamol, Salicylsäure und -derivate wie Acetylsalicylsäure, Diclophenac, Ibuprofen, Ketoprofen, Naproxen etc. , Antiphlogistika, Vasodilatatoren, Diuretica, Gichtmittel, Zytostatika, Muskelrelaxantien, Kontrazeptiva z.B. in Form von Hormonpflastern, Suchtentwöhnungsmittel in Form von beispielsweise Nicotinpflastern, Pflanzenextrakte, Provitamine wie z.B. Beta-Carotin, Vitamine wie z. B. Vitamin C, A, B, E etc., über eine transdermale Applikation in einer erfindungsgemäßen Zusammensetzung z.B. in Form eines transdermalen Wirkstoff-Patches zu verabreichen.

Besonders bevorzugte pharmazeutische Wirkstoffe, sind solche die ausgewählt sind aus der Gruppe der antibakteriellen Mittel wie insbesondere Silberverbindungen oder anderer bakteriostatischer/bakteriozider Substanzen wie beispielsweise Octinidin, PVP-Iod, etc. zur Behandlung von chronischen Wunden oder sogenannten Problemwunden.

Auch die Hydrokolloide, insbesondere solche auf Basis natürlicher Polymere wie Polysaccharide können gewisse therapeutische Wirkungen aufweisen. So wirkt das bevorzugt verwendete Hydrokolloid (Natrium-)Alginat in gewissem Ausmaß antiviral und Hyaluronsäure wird eine gewisse Wirkung in der Re-Epithelisierung und als Antioxidans und Feuchtigkeitsspender in der Hautpflege nachgesagt, sie sind jedoch keine Wirkstoffe im Sinne der Erfindung.

Die erfindungsgemäßen gefriergetrockneten Zusammensetzungen können weiterhin gegebenenfalls einen oder mehrere Hilfsstoffe enthalten. Hilfsstoffe schließen ein: pH-Einstellungsmittel, wie Pufferstoffe, anorganische und organische Säuren oder Basen; Fettsubstanzen, wie Mineralöle, wie Paraffinöle oder Vaselineöle, Siliconöle, Pflanzenöle wie Kokosöl, Süßmandelöl, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamonöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Neutralöl, Squalan, Fettsäureester, Ester von Fettalkoholen wie Triglyceride, und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle (sogenannte kosmetische Öle), wie beispielsweise in der CTFA-Abhandlung, Cosmetic Ingredient Handbook, 1. Auflg., 1988, The Cosmetic, Toiletry and Fragrance Association, Inc., Washington, erwähnt, oberflächenaktive Mittel neben den oben erwähnten Waschtensiden, wie Dispergiermittel, Netzmittel, Emulgatoren etc.; Füllstoffe; Stabilisatoren; Cosolventien; pharmazeutisch und kosmetisch gebräuchliche oder sonstige Farbstoffe und Pigmente, insbesondere solche, die primär zur farblichen Gestaltung der Hydrogel-Zusammensetzung eingesetzt werden und nicht zur Applikation und farblichen Gestaltung am menschlichen Körper, wie solche Pigmente und Farbstoffe wie die unter der Gruppe der Wirkstoffe aufgeführten dekorativen Farbstoffe; Konservierungsmittel; Weichmacher; Schmiermittel bzw. Gleitmittel; etc.

Ein besonders bevorzugter Hilfsstoff ist Kalilauge (KOH) zur Einstellung des bevorzugten pH-Werts zwischen pH 3,0 und 6,5, bevorzugt zwischen pH 4,0 und 6,0, bevorzugter zwischen pH 4,5 und 5,5, besonders bevorzugt pH 5.0.

Weitere erfindungsgemäß bevorzugte Hilfsstoffe sind Fette und Öle. Dabei sind insbesondere kosmetische Öle wie vorstehend aufgezählt, insbesondere Triglyceride, besonders bevorzugt Capryl/Capronsäure-Triglyceride, Squalan und/oder Jojobaöl bevorzugt.

Ein weiterer bevorzugter Hilfsstoff ist ausgewählt aus der Gruppe der Füllstoffe, wobei besonders bevorzugt Mannitol enthalten ist

Generell schließt die Einordnung der vorstehend erwähnten Stoffe in die Kategorie der Hilfsstoffe im Rahmen der vorliegenden Erfindung nicht aus, dass diese Hilfsstoffe auch gewisse kosmetische und/oder therapeutische Wirkungen entfalten können, was in besonderem Maß für die genannten bevorzugt eingesetzten kosmetischen Öle gilt. Hilfsstoffe können den erfindungsgemäßen Zusammensetzungen in Mengen bis zu 50 Gew.-% bezogen auf die gefriergetrocknete Gesamtzusammensetzung des Endproduktes hinzugefügt werden. Gegenstand der Erfindung ist außerdem ein Verfahren zur Herstellung von gefriergetrockneten Zusammensetzungen, das die folgenden Schritte umfasst:

a) Herstellen einer wässrigen Suspension oder einer Lösung mindestens eines natürlichen Polymers
b) gegebenenfalls Einmischen mindestens eines weiteren Polymers, das von dem in Schritt a) oder e) verwendeten Polymer verschieden ist
c) gegebenenfalls Einmischen eines oder mehrerer Wirk- und/oder Hilfsstoffe
d) Einstellen des pH Werts der wässrigen Suspension oder Lösung auf einen pH-Wert von pH 3,0 bis pH 6,5
e) Herstellen einer wässrigen Suspension oder einer Lösung eines Polymers auf Basis von Polyacrylsäuren und Salzen davon und Einstellen des pH Wertes der Lösung mit Laugen auf einen pH-Wert zwischen pH 3,0 und pH 6,5
f) Vereinigung der unter a)-d) und der unter e) hergestellten Suspensionen oder Lösungen und gegebenenfalls Einstellung des pH-Wertes auf pH 3,0 bis 6,5
g) Giessen oder Ausstreichen der Mischung in eine geeignete Form oder auf eine geeignete Fläche
h) Gefrieren der Mischung und
i) Gefriertrocknung der Mischung unter Bildung der gefriergetrockneten Zusammensetzung.

Gegebenenfalls können zwischen diesen Schritten weitere Schritte durchgeführt werden, insbesondere ist es möglich, nach dem Schritt h) die gefrorenen Zusammensetzungen aus der gegebenenfalls verwendeten Form zu entnehmen.

Im Anschluss an das oben beschriebene Herstellungsverfahren können die gefriergetrockneten Zusammensetzungen insbesondere durch Schneiden und Stanzen in die gewünschte Form überführt werden. Insbesondere ist es möglich, durch Zuschneiden der nach dem genannten Verfahren erhältlichen gefriergetrockneten Zusammensetzungen in nahezu beliebiger Form bereitzustellen. Insbesondere ist es möglich durch das Zuschneiden sowohl die Dicke der Zusammensetzungen als auch die geometrische Form anzupassen.

Um Zusammensetzungen in Form von flächigen Ausgestaltungen wie z. B. Sheets, Matrices, Auflagen, Pads, Vliesen, Masken, Blättern, Lagen, Schichten oder in anderen denkbaren flächigen Formen zu erhalten, werden die gefriergetrockneten Zusammensetzungen die nach dem oben beschriebenen Verfahren erhältlich sind, vorzugsweise auf eine Dicke von 0,5 mm bis 2 cm zugeschnitten.

Des weiteren werden bevorzugt Zuschnitte gewählt, in denen Länge und Breite der gefriergetrockneten Zusammensetzung wenigstens 10-mal, bevorzugt wenigstens 20-mal so groß wie die Dicke sind.

Es ist auch möglich, Zuschnitte bereitzustellen, die in ihrer geometrischen Form z. B. an das zu behandelnde Körperareal angepasst sind oder solche, die Phantasieformen aufweisen. So können die erfindungsgemäßen gefriergetrockneten Zusammensetzungen insbesondere für die kosmetische Anwendung Zuschnitte in Form eines Gesichts oder anderer zu behandelnder Körperpartien aufweisen. In einem solchen Fall handelt es sich um erfindungsgemäße Zusammensetzungen in Form von Masken.

Es ist jedoch auch möglich, nach dem oben beschriebenen Verfahren gefriergetrocknete Zusammensetzungen in Form großformatiger, mechanisch stabiler Formkörper zu erhalten, wobei die Ausgestaltung solcher Formkörper maßgeblich durch die Wahl der Form, in der die Zusammensetzungen gefroren werden, bestimmt wird.

Für die therapeutische Anwendung ist es insbesondere vorteilhaft, Sheets oder Pads in Form von rechteckigen Stücken zuzuschneiden. Solche Rechtecke können je nach Größe des zu behandelnden Areals Flächen von bevorzugt mindestens etwa 25 $cm^2$, bevorzugter von mindestens etwa 50 $cm^2$, noch bevorzugter von mindestens etwa 100 $cm^2$ aufweisen.

Dabei ist die Größe, die Fläche sowie die Dicke der gefriergetrockneten Zusammensetzung unter anderem durch die gewünschte Applikationsform oder den Ort der Anwendung bestimmt. So ermöglicht bei der äußeren kosmetischen oder pharmazeutischen Verwendung die Applikation auf größeren Körperflächen oder den Haaren (z.B. der direkte Auftrag der rehydratisierten Zusammensetzung auf dem Rücken etc., oder der Einsatz als Badezusatz) den Einsatz größerer Ausgestaltungen der Zusammensetzungen, wohingegen bei der Anwendung auf kleineren Körperpartien (z.B. Wange etc.) kleinere Ausgestaltungen der Zusammensetzungen bevorzugt sind. Auch für die therapeutische Behandlung ist eine Größenanpassung der Zusammensetzung z. B. an das räumliche Ausmaß einer Wunde von Bedeutung.

Die nach dem beschriebenen Verfahren erhältlichen Hydrogel-Zusammensetzungen können außerdem gestanzt oder mit einer Prägung versehen werden.

Bevorzugt weisen flächige Ausgestaltungen der erfindungsgemäßen gefriergetrockneten Zusammensetzung eine Fläche von 5 bis 500 $cm^2$ auf, wobei diese Fläche sich aus den beiden längsten Seitenlängen solcher flächigen Zusammensetzungen ergibt.

Zweckmäßig geht man bei der Herstellung so vor, dass man zunächst eine wässrige Lösung des natürlichen Polymers herstellt und anschließend die gegebenenfalls zuzusetzenden weiteren Polymere aus der Gruppe der natürlichen, modifizierten natürlichen oder synthetischen Polymere hinzufügt. Diese können entweder direkt in die wässrige Polymer-Lösung eingemischt und darin gelöst werden oder es werden wässrige Lösungen oder Suspensionen dieser weiteren Polymere hergestellt, die dann mit der Lösung oder Suspension des natürlichen Polymers vermischt wird. In diese so erhältliche Polymerlösung oder -suspension werden gegebenenfalls der oder die Wirkstoffe sowie gegebenenfalls ein oder mehrere Hilfsstoffe hinzugegeben und vermischt. Nach dem gründlichen Vermischen aller Bestandteile wird der pH-Wert der Zusammensetzung gegebenenfalls durch Zugabe eines Hilfsstoffs aus der Gruppe der pH-Einstellungsmittel, bevorzugt mit einer anorganischen Lauge wie vorzugsweise einer Kaliumhydroxid-Lösung auf einen pH-Wert zwischen pH 3,0 und 6,5, bevorzugt zwischen pH 4,0 und 6,0, bevorzugter zwischen pH 4,5 und 5,5, besonders bevorzugt pH 5.0 eingestellt.

Anschließend wird der Mischung mindestens ein Polymer auf Basis von Polyacrylsäuren und Salzen davon, bevorzugt ein Carbomer zugesetzt. Dies erfolgt durch Herstellung einer mit einem pH-Einstellungsmittel wie insbesondere einer Alkalimetall-Lauge wie bevorzugt mit Kaliumhydroxid teilneutralisierten Polyacrylsäure- bzw. Carbomerlösung, welche anschließend durch Rühren in die schon hergestellte Lösung des natürlichen Polymers und der gegebenenfalls hinzugefügten weiteren Stoffe, eingearbeitet wird.

Werden für die Herstellung der Lösung oder Suspension, die der Gefriertrocknung unterworfen wird, öllösliche Wirkstoffe verwendet, so werden diese bevorzugt in gegebenenfalls als Hilfsstoffe verwendeten Ölen (insbesondere Squalan, Jojobaöl und/oder Triglyceriden wie Neutralöl) gelöst und anschließend der Lösung des natürlichen Polymers sowie gegebenenfalls weiterer Polymere und Wirk- und/oder Hilfsstoffe zugesetzt. Diese Herstellweise besitzt den Vorteil, dass sich kurzzeitig stabile Lösungen bzw. Suspensionen bilden. Es werden keine Emulgatoren oder oberflächenaktive Substanzen wie z. B. Tenside benötigt, und es findet während der Verarbeitung keine Phasentrennung der Lösung oder Suspension bei Verwendung öllöslicher bzw. öliger Hilfs- bzw. Wirkstoffe statt. Bevorzugt werden jedoch wasserlösliche Wirkstoffe verwendet.

Damit die gefriergetrockneten Zusammensetzungen nach der Gefriertrocknung eine ausreichende mechanische Stabilität insbesondere für das Zuschneiden und/oder Stanzen aufweisen, ist es erforderlich, dass die wässrige Lösung bzw. Suspension der erfindungsgemäßen Polymer-Zusammensetzung eine gewisse Polymer-Konzentration, insbesondere des Polymers aus der Gruppe der natürlichen Polymere und der Polyacrylsäure bzw. -derivate aufweist. Die jeweilige genaue Konzentration hängt natürlich von der Art der verwendeten Polymere ab. Sie beträgt zweckmäßig etwa mindestens 0,5 Gew.-% bezogen auf die Gesamtmenge der Lösung oder Suspension die der Gefriertrocknung unterworfen wird, bevorzugt mindestens etwa 1,0 Gew.-% bis zu mindestens etwa 1,5 Gew.-%, bevorzugt weniger als 10 Gew.-%, noch bevorzugter weniger als 5 Gew.-% (Gewicht der Polymere aus der Gruppe der natürlichen und ggf. modifizierten natürlichen und/oder weiteren synthetischen Polymere plus Gewicht der Polyacrylsäuren, bezogen auf das Gesamtgewicht der Lösung).

Die so hergestellte Lösung oder Suspension wird dann entweder in geeignete Formen oder auf einer Fläche ausgegossen und gefroren. Das Abkühlen bzw. Gefrieren der Lösung oder Suspension kann an sich in beliebiger Weise erfolgen wie beispielsweise durch Anblasen mit kalter Luft, Abkühlen durch Aufbringen auf eine mit Kühlsole durchflossene Platte oder auch das Eintauchen der Formen in flüssige Gase, wie z.B. das Eintauchen in flüssigen Stickstoff. Die Abkühlgeschwindigkeit beeinflusst dabei die Größe der gebildeten Eiskristalle. Diese beeinflussen wiederum die Porengrößenverteilung des gebildeten gefrorenen Hydrogels. Werden wenige große Kristalle gebildet, so weist die gefrorene Zusammensetzung wenige große Poren auf, werden viele kleine Kristalle gebildet, weist es viele kleine Poren auf. Die Kristalle werden um so kleiner, je höher die Abkühlgeschwindigkeit der Lösung bzw. Suspension ist. Dabei wird eine Einfriergeometrie bevorzugt, bei der die Zusammensetzungen bei mindestens < -20 °C auf einer kalten Platte eingefroren wird.

Die Gefriertemperatur, die erforderlich ist, hängt unter anderem davon ab, wie stark die Gefrierpunktserniedrigung durch die in der Lösung enthaltenden Wirkstoffe bzw. Hilfsstoffe ist. Zweckmäßig liegt die Temperatur unterhalb des Gefrierpunktes von Wasser bis zur Temperatur von flüssigem Stickstoff (- 196°C). Bevorzugt ist die Gefriertemperatur etwa -10 bis -80°C, besonders bevorzugt -20 bis - 60 °C

Anschließend werden die gefrorenen Zusammensetzungen der Gefriertrocknung unterworfen. Die Gefriertrocknung kann in an sich bekannter Weise nach allgemein bekannten Gefriertrocknungsverfahren, wie z.B. auch in der

DE 4328329 C2, der DE 4028622 C2 oder der DE 10350654 A1 beschrieben, erfolgen.

Die Menge der in der gefrierzutrocknenden Lösung bzw. Suspension enthaltenen Feststoffe wie natürliche, modifizierte natürliche oder synthetische Polymere, Wirkstoffe und Hilfsstoffe beeinflusst maßgeblich die Dichte (Gewicht der gefriergetrockneten Zusammensetzung bezogen auf das Volumen der geometrischen Form derselben) der erhaltenen gefriergetrockneten Zusammensetzung. Die Dichte ist wiederum eine wichtige Größe für die Porosität der gefriergetrockneten Zusammensetzung und damit wiederum für deren Auflösungsgeschwindigkeit bzw. Quellfähigkeit beim Befeuchten mit Wasser, einer Wirk- und/oder Hilfsstofflösung und/oder mit Körper- bzw. Wundflüssigkeiten. Die poröse Struktur gefriergetrockneter Zusammensetzungen ist eine wesentliche Grundlage für die schnelle Feuchtigkeitsaufnahme und gute Rehydratation, da durch die große Oberfläche im porösen Material ein inniger Austausch zwischen wässriger Phase und fester Zusammensetzung während des Rehydratisierungsprozesses stattfinden kann. Je höher die Konzentration Polymere sowie ggf. Wirkstoffe und Hilfsstoffe in der Lösung ist, umso höher wird die Dichte und damit umso geringer der Porositätsgrad der gefriergetrockneten Zusammensetzung und umgekehrt. Allerdings hängt der Porositätsgrad der gefriergetrockneten Zusammensetzungen nicht allein von der Materialdichte ab. Vielmehr ist die Materialporosität im wesentlichen eine Funktion zweier Parameter, der Materialdichte und der Eiskristallgröße. Hohe Feststoffgehalte in der wässrigen Suspension erhöhen die Materialdichte im gefriergetrockneten Endprodukt und verringern die Kontaktfläche Rehydratisierungsmittel/Feststoff. Hohe Einfriergradienten führen zu kleinen Eiskristallen, welche zu großen inneren Materialoberflächen führen, was wiederum die Rehydratisierung begünstigt. Zur schnellen Befeuchtung und Auflösung bzw. Quellung der gefriergetrockneten Zusammensetzungen sind also niedrige Materialdichten und kleine Eiskristalle vorteilhaft.

Unter dem Gesichtspunkt Dichte/Porositätsgrad bzw. der Auflösungs- oder Rehydratisierungsgeschwindigkeit wird die Rezepturierung und Herstellung der erfindungsgemäßen Zusammensetzungen so ausgerichtet, dass die Dichten der damit erhältlichen Zusammensetzungen zweckmäßig bei etwa 0,01 g/cm$^3$ bis zu 0,8 g/cm$^3$, bevorzugt etwa 0,015 g/cm$^3$ bis zu 0,5 g/cm$^3$.bevorzugt etwa 0,02 g/cm$^3$ bis zu 0,1 g/cm$^3$ liegen. Der Begriff der Dichte, wie er vorliegend verwendet wird, bezeichnet das Gewicht der gefriergetrockneten Zusammensetzung bezogen auf das Volumen der äußeren geometrischen Form derselben. Das Gewicht der einzelnen, zugeschnittenen gefriergetrockneten Zusammensetzungen hängt dabei natürlich von deren Größe ab und liegt je Dosierungseinheit, also je Zuschnitt für die letztendlich vorgesehene Anwendung im allgemeinen bei etwa 10 mg bis 6 g, bevorzugt bei 20 mg bis 1000 mg, noch bevorzugter bei 50 mg bis 500 mg bei Flächen von etwa 5 bis 500 cm$^2$, bevorzugt mindestens 10 cm$^2$, bevorzugter von mindestens etwa 15 cm$^2$, noch bevorzugter von mindestens etwa 20 cm$^2$ und Dicken von etwa 0,5 mm bis 2 cm.

Insbesondere auch aus ästhetischen Gründen, besonders in der kosmetischen, aber auch in der therapeutischen Anwendung sind solche Zusammensetzungen erwünscht, die außerdem über eine hohe optische Dichte verfügen. Dabei bezeichnet optische Dichte die quantitative Einheit optische Dichte, gemessen als dekadischer Logarithmus des Quotienten von durchgelassener Lichtintensität zu eingestrahlter Lichtintensität, ermittelt mit einem Heiland SW Densitometer TD 03 an gefriergetrockneten Zusammensetzungen mit einer Schichtdicke von 1 mm. Die gefriergetrockneten Zusammensetzungen der vorliegenden Erfindung weisen bevorzugt eine optische Dichte von $\geq$ 0,02, bevorzugter $\geq$ 0,03, noch bevorzugter $\geq$ 0,05 pro mm Schichtdicke auf.

Aufgrund des vorstehend beschriebenen Einflusses des pH-Wertes der erfindungsgemäßen Zusammensetzungen auf die zu erzielende optische Dichte erfolgt die Herstellung und Rezepturierung, insbesondere die Einstellung des pH-Wertes derart, dass möglichst hohe optische Dichten, vorzugsweise optische Dichten $\geq$ 0,02, bevorzugter $\geq$ 0,03, noch bevorzugter $\geq$ 0,05 pro mm Schichtdicke erzielt werden.

Die erfindungsgemäßen gefriergetrockneten Zusammensetzungen sind bei Flüssigkeitszufuhr unter Bildung eines homogenen, feindispersen Gels schnell und nahezu vollständig rehydratisierbar, wobei sich insbesondere hydrophile bzw. wässrige Flüssigkeiten zu einer schnellen Rehydratisierung eignen. Die erfindungsgemäß eingesetzten Polymere sind nicht im eigentlichen Sinne löslich, dahingehend, daß die Feststoffbestandteile vollständig in einem Lösungsmittel aufgelöst und homogen darin verteilt werden, als vielmehr eine Quellung und Rehydratisierung der Polymere unter Einlagerung hoher Mengen Wasser und Ionen in das Polymergerüst der Hydrokolloide erfolgt.

Nach der Rehydratisierung und im gequollenen Zustand ist die erfindungsgemäße rehydratisierte Zusammensetzung dabei speziell in Hinblick auf das gewünschte schnelle und rückstandsfreie bzw. einheitliche und homogene Auflösungs- bzw. Rehydratisierungsverhalten und die gute Verträglichkeit frei von sichtbaren, makroskopischen Partikeln oder Faserbestandteilen wie z.B. textilen Rayon- (Viskose-) oder Baumwollfasern oder von nicht-quellenden synthetischen Polymerfasern z. B. auf Basis von Polyamid, Polyester oder Polyether. Die gebildeten Gele sind dabei insbesondere frei von solchen sichtbaren, makroskopischen Bestandteilen mit einer Größe > 5 mm. Somit sind erfindungsgemäß bevorzugt gefriergetrocknete Zusammensetzungen, die keine textilen Faserbestandteile enthalten.

Desweiteren sind die erfindungsgemäßen gefriergetrockneten Zusammensetzungen nicht chemisch vernetzt und somit frei von chemischen Vernetzungsmitteln.

Bevorzugt weisen die erfindungsgemäßen gefriergetrockneten Zusammensetzungen bei Rehydratisierung von 3 g der Zusammensetzung in 97 g destilliertem Wasser bei einer Temperatur von 20-25 °C und einem pH-Wert von pH von 4,0 bis 6,0 eine Viskosität von 10 bis 200 mPas auf. Die Viskositätsmessung erfolgt dabei mit dem Viskotester VT

2 plus mit der Spindel Nr. 1 von der Fa. Haake.

Die erfindungsgemäßen gefriergetrockneten Zusammensetzungen zeichnen sich durch ihre besondere Benetzungsgeschwindigkeit sowie ihre besondere Absorptions-, Flüssigkeitsaufnahme- und -speicherkapazität aus. Insbesondere zeichnen sich die erfindungsgemäßen gefriergetrockneten Zusammensetzungen durch ihre besondere Aufnahme- und Speicherkapazität von physiologischen Flüssigkeiten aus.

Die Flüssigkeitsaufnahme- bzw. -speicherkapazität der erfindungsgemäßen gefriergetrockneten Zusammensetzungen bezeichnet dabei die Fähigkeit zur Aufnahme von Flüssigkeitsmengen insbesondere in Kombination mit der Fähigkeit diese aufgenommenen Flüssigkeitsmengen zu speichern und zu halten. Dabei sind erfindungsgemäß solche gefriergetrockneten Zusammensetzungen bevorzugt, die in der Lage sind Flüssigkeitsmengen des 1 bis 200fachen, bevorzugt des 10 bis 100fachen ihres Eigengewichtes aufzunehmen und zu speichern.

$$Q_M = \frac{m_{Gel}}{m_{tr.Pr.}}$$

Hierzu bezeichnet der Massenquellungsgrad ($Q_M$) die Menge Flüssigkeit, die durch die erfindungsgemäße Zusammensetzung aufgenommen werden kann. $Q_M$ bezeichnet dabei das Verhältnis der Masse der gequollenen Zusammensetzung ($m_{Gel}$) zur Masse der trockenen Zusammensetzung vor der Quellung ($m_{tr.Pr.}$).

Zur Messung des Massenquellungsgrades wird die gefriergetrocknete Zusammensetzung somit gewogen und anschließend in eine Schale mit einem Überschuss an destilliertem Wasser mit einer Temperatur von 15 - 25 °C auf die Wasseroberfläche gelegt und für 10 Minuten quellen gelassen. Das überschüssige Wasser wird ohne mechanische Einwirkung abgegossen. Nach erneuter Gewichtsmessung der gequollenen Zusammensetzung wird der Massenquellungsgrad gemäß vorstehender Formel ermittelt.

Die erfindungsgemäßen gefriergetrockneten Zusammensetzungen weisen bevorzugt einen Massenquellungsgrad von 15 - 100 auf.

Es ist außerdem möglich, das Flüssigkeitshaltevermögen bezogen auf das Gewicht der Zusammensetzung anzugeben. Dazu wird die im vorstehend beschriebenem Versuchsaufbau ermittelte Gewichtszunahme der gequollenen Materialproben, nachdem die überschüssige Flüssigkeit abgegossen wurde, auf das dieser Gewichtszunahme entsprechende Flüssigkeitsvolumen umgerechnet und dieses aufgenommene Flüssigkeitsvolumen bezogen auf 1 g der eingesetzten Zusammensetzung angegeben.

Erfindungsgemäß ist auch eine Mehrzahl der genannten gefriergetrockneten Zusammensetzungen oder -Zuschnitte in einem Behältnis umfasst. Auch kann es sich um Mischungen von Zuschnitten verschiedener Geometrien oder unterschiedlicher Größen z. B. zur parallelen Behandlungen verschiedener Körperpartien handeln. Die Zuschnitte können einzeln abgepackt sein, was insbesondere in der therapeutischen bzw. pharmazeutischen Anwendung bevorzugt ist. Zuschnitte für die kosmetische Anwendung liegen bevorzugt in einer Mehrzahl neben- oder übereinander in Kontakt in einem geeigneten Behältnis oder einer geeigneten Verpackung vor.

Die erfindungsgemäßen gefriergetrockneten Zusammensetzungen dienen der äußeren kosmetischen sowie der äußeren und transdermalen pharmazeutischen Anwendung bei Menschen oder Tieren. Die äußere Anwendung erfolgt dabei insbesondere bei der kosmetischen Applikation so, dass die erfindungsgemäße Zusammensetzung mit Wasser bzw. einer wässrigen Lösung, die einen oder mehrere Wirkstoffe und/oder einen oder mehrere Hilfsstoffe enthält, angefeuchtet und unter Bildung eines homogenen Gels gelöst bzw. rehydratisiert wird, wobei dieses Gel frei ist von sichtbaren, makroskopischen Partikeln oder Faserbestandteilen wie insbesondere stabilisierenden textilen oder nichtquellbaren Faserbestandteilen.

Wird die erfindungsgemäße gefriergetrocknete Zusammensetzung in einer größeren Wassermenge gelöst, handelt es sich in der Regel um eine Badeanwendung und diese Anwendung ist erfindungsgemäß in der äußeren Anwendung enthalten.

Es ist jedoch auch möglich, die erfindungsgemäßen gefriergetrockneten Zusammensetzungen trocken auf die zu behandelnde Körperpartie aufzubringen, dort mit Wasser oder einer wässrigen Lösung eines oder mehrerer Wirkstoffe und/oder Hilfsstoffe oder einer physiologischen Lösung anzufeuchten und unter leichter mechanischer Einwirkung, insbesondere durch Massieren aufzulösen und zu verteilen.

Gegebenenfalls können nach der Behandlung verbleibende Gel-Rückstände durch Abwaschen, -wischen, -spülen oder Abreiben von der behandelten Körperpartie entfernt werden. Diese Art der Anwendung ist besonders bevorzugt bei Verwendung der erfindungsgemäßen gefriergetrockneten Zusammensetzungen als kosmetische Maske.

Insbesondere in der therapeutischen Anwendung, besonders bei Verwendung der erfindungsgemäßen gefriergetrockneten Zusammensetzungen als Wundauflage z. B. bei chronischen Wunden, Ulcus cruris, Dekubitus etc., sowie insbesondere bei Verwendung der erfindungsgemäßen gefriergetrockneten Zusammensetzungen zum Wundexsudatmanagement können die gefriergetrockneten Zusammensetzungen auch trocken in die Wunde eingebracht werden, wo sie aufgrund ihres hohen Absorptionsvermögens und ihrer besonderen Flüssigkeitsaufnahme- und -speicherkapazität,

insbesondere für physiologische Flüssigkeiten, überschüssiges Wundexsudat bzw. Wundflüssigkeit aufnehmen können, um dadurch unterstützend in der Heilung der Wunde zu wirken.

Dabei wird in der Regel die trocken in eine zu behandelnde Wunde auf- bzw. eingebrachte gefriergetrocknete Zusammensetzung in der Wunde durch die vorhandenen Körper- bzw. Wundflüssigkeiten rehydratisiert und im Lauf der weiteren Behandlung entweder vollständig in der Wunde resorbiert oder mit der Wundflüssigkeit oder anderen Wundbehandlungsflüssigkeiten wieder ausgespült.

Die vorliegende Erfindung betrifft auch eine Kombination, enthaltend mindestens eine der erfindungsgemäßen gefriergetrockneten Zusammensetzungen bzw. deren Zuschnitte sowie mindestens eine wässrige Lösung, die einen oder mehrere Wirkstoffe und/oder mindestens einen oder mehrere Hilfsstoffe enthält (eine sogenannte Aktivatorlösung), in einer zusammengehörenden, räumlichen Anordnung (Anwendungspaket, Set, Kit-of-Parts etc.).

Bei der Wirkstoff- bzw. Aktivatorlösung kann es sich z.B. um Lösungen von leicht-flüchtigen Wirk- und/oder Hilfsstoffen handeln, die aufgrund des Herstellverfahrens durch die Gefriertrocknung nicht in die gefriergetrocknete Zusammensetzung eingebracht werden sollen bzw. können, wie z.B. gewisse Anteile ätherischer Öle, Parfums etc. Auch können solche Wirk- und/oder Hilfsstoffe enthalten sein, die eine befeuchtende Wirkung erzielen, die insbesondere bei der äußerlichen Anwendung auf der Haut erwünscht und bevorzugt ist, und die aufgrund dieser befeuchtenden Wirkung oder aufgrund von Hygroskopie-Neigungen nicht oder nur in geringen Mengen in die erfindungsgemäßen gefriergetrockneten Zusammensetzungen eingearbeitet werden können, da dadurch die Stabilität eventuell enthaltener feuchtigkeitslabiler Wirkstoffe nicht mehr aufrechterhalten werden kann. Auch die Verwendung von physiologischen Lösungen ist bevorzugt, da durch solche Elektrolyte in die rehydratisierte Zusammensetzung eingebracht werden können, die aufgrund der oben genannten Polymereigenschaften nicht oder nur bedingt in die gefrierzutrocknenden Polymermischungen eingebracht werden können.

Die Ausgestaltung solcher Kit-of-parts Kombinationen aus erfindungsgemäßer gefriergetrockneter Zusammensetzung einerseits und Wirkstofflösung andererseits kann dabei vorsehen, dass die beiden Bestandteile separat aus der Kit-of-parts Anordnung entnommen werden und außerhalb davon für die weitere Verwendung zusammengeführt und aufgelöst werden. Es ist jedoch auch denkbar, dass eine Zusammenführung der beiden Komponenten innerhalb der Kit-of-parts-Verpackung selbst erfolgt und die rehydratisierte Zusammensetzung sodann aus dieser direkt der weiteren kosmetischen oder pharmazeutischen äußerlichen oder transdermalen Verwendung zugeführt wird. Dies kann bevorzugt direkt durch den Endverbraucher durchgeführt werden.

Die erfindungsgemäßen gefriergetrockneten Zusammensetzungen enthalten im trockenen Endprodukt ≥ 5 Gew.-%, bevorzugt ≥10 Gew.-%, noch bevorzugter ≥15 Gew.-% eines oder mehrerer Polymere auf Basis von Polyacrylsäuren und Salzen davon. Besonders bevorzugt sind solche aus der Gruppe der Carbomere ausgewählt.

Die erfindungsgemäßen gefriergetrockneten Zusammensetzungen enthalten im trockenen Endprodukt außerdem ≥25 Gew.-%, bevorzugt ≥40 Gew.-%, noch bevorzugter ≥50 Gew.-% eines oder mehrerer natürlicher Polymere. Besonders bevorzugt sind solche aus der Gruppe der anionischen natürlichen Polymere, insbesondere aus der Gruppe der hydrokolloid-bildenden Polysaccharide wie insbesondere aus der Gruppe der Alginate ausgewählt.

Die erfindungsgemäßen gefriergetrockneten Zusammensetzungen können darüber hinaus außerdem ≥ 5 Gew.-%, bevorzugt ≥ 10 Gew.-%, noch bevorzugter ≥ 15 Gew.-% eines oder mehrerer weiterer, von den vorstehend genannten unterschiedliche Polymere enthalten. Besonders bevorzugt sind solche weiteren Polymere aus der Gruppe der Carrageene oder aus der Gruppe der Mucopolysaccharide wie Hyaluronsäure oder aus den Collagenen.

Weiterhin können die erfindungsgemäßen gefriergetrockneten Zusammensetzungen ≥ 0,5 Gew.-%, bevorzugt ≥ 2,0 Gew.-%, noch bevorzugter ≥ 5,0 Gew.-% eines oder mehrerer Wirkstoffe enthalten. Besonders bevorzugt sind solche Wirkstoffe aus der Gruppe der Ascorbinsäure und ihrer Derivate, wie insbesondere Ascorbylglucosid und/oder Magnesium-Ascorbylphosphat ausgewählt.

Auch ist es möglich, daß die erfindungsgemäßen gefriergetrockneten Zusammensetzungen ≥ 5 Gew.-%, bevorzugt ≥ 10 Gew.-%, noch bevorzugter ≥ 20 Gew.-% eines oder mehrerer gegebenenfalls weiterer Hilfsstoffe enthalten.

Besonders bevorzugt sind hierbei aus der Gruppe der Hilfsstoffe solche aus der Gruppe der kosmetischen Öle, wie Triglyceride, insbesondere Capryl-/Capronsäure-Triglyceride (Neutralöl), Jojobaöl oder Squalan, sowie aus der Gruppe der Füllstoffe insbesondere bevorzugt Mannitol ausgewählt.

Ein weiterer besonders bevorzugter Hilfsstoff ist ausgewählt aus der Gruppe der pH-Einstellungsmittel, wie wässrige Lösungen von Triethanolamin und/oder Alkalilaugen, insbesondere Alkalilaugen, wobei insbesondere wässrige Lösungen von Kaliumhydroxid bevorzugt sind. Solche Laugen werden den erfindungsgemäßen gefriergetrockneten Zusammensetzungen zur Einstellung des pH-Wertes der gefriergetrockneten Zusammensetzungen - auf einen pH-Wert zwischen pH 3,0 und 6,5, bevorzugt zwischen pH 4,0 und 6,0, bevorzugter zwischen pH 4,5 und 5,5, besonders bevorzugt pH 5,0 zugesetzt. Weist die Zusammensetzung aus Polymeren, Wirkstoffen, und gegebenenfalls Hilfsstoffen per se bereits einen erfindungsgemäß gewünschten pH-Wert auf, so kann auf die Zugabe von pH-Einstellungsmitteln selbstverständlich verzichtet werden.

Die angegebenen Mengen beziehen sich jeweils auf das Gesamtgewicht der gefriergetrockneten Zusammensetzung.

Die erfindungsgemäßen gefriergetrockneten Zusammensetzungen enthalten gegebenenfalls auch Reste von Wasser. Da die Vorteile der erfindungsgemäßen gefriergetrockneten Zusammensetzungen insbesondere in der hohen Stabilität gegen Verderb und Inaktivierung von darin gegebenenfalls enthaltenen instabilen Wirkstoffen besteht, ist dieser sogenannte Restwassergehalt so gering wie möglich zu halten. Je nach Zusammensetzung kann der Wassergehalt in der gefriergetrockneten Zusammensetzungen bei bis zu 15 Gew.-%, bezogen auf die Gesamtzusammensetzung, liegen. Der Wassergehalt kann sich nach der Herstellung der gefriergetrockneten Zusammensetzungen durch Gefriertrocknung bei der Lagerung verändern, in der Regel erhöhen. Bevorzugt liegt der Wassergehalt nach der Gefriertrocknung bei maximal 10 Gew.-%, bevorzugt bei weniger als 5 Gew.-%, bevorzugter bei weniger als 1 Gew.-%.

Eine besonders bevorzugte gefriergetrocknete Zusammensetzung enthält:

- ≥15 Gew.-% eines oder mehrerer Carbomere, wie z. B. Carbopol Ultrez 20
- ≥50 Gew.-% eines oder mehrerer Alginate, wie z. B. Natriumalginat,
- ≥15 Gew.-% eines oder mehrerer weiterer natürlicher Polymere, wie z.B. Carrageen, und/oder
- ≥2,5 Gew.-% Hyaluronsäure, sowie

≤ 10 Gew.-%, bevorzugt ≤5 Gew.-%, bevorzugter ≤1 Gew.-% Wasser, mit der Maßgabe, dass eine 1 gewichtsprozentige Lösung oder Suspension einer solchen gefriergetrockneten Zusammensetzung in Wasser bei 20 °C einen pH Wert zwischen pH 4,0 und pH 6,0 und einen Massenquellungsgrad von 20 bis 60 aufweist.

Eine weitere bevorzugte Ausführungsform enthält darüber hinaus ≥ 5,0 Gew.-% eines oder mehrerer Wirkstoffe, insbesondere Ascorbinsäure (Vitamin C) oder seine Derivate oder Salicylsäure oder seine Derivate wie Acetylsalicylsäure (ASS) oder Silberverbindungen wie Silbernitrat und andere in der Wundbehandlung gebräuchliche Wirkstoffe,
≤ 20 Gew.-% eines oder mehrerer Hilfsstoffe, wie insbesondere kosmetische Öle wie Neutralöl, Jojobaöl oder Squalan, mit der Maßgabe, dass eine 1 gewichtsprozentige Lösung oder Suspension einer solchen gefriergetrockneten Zusammensetzung in Wasser bei 20 °C einen pH Wert zwischen pH 4,0 und pH 6,0 und einen Massenquellungsgrad von 20 bis 100 aufweist.

Eine bevorzugtere gefriergetrocknete Zusammensetzung enthält:

- ≥5 Gew.-% eines oder mehrerer Carbomere, wie z. B. Carbopol Ultrez 20
- ≥15 Gew.-% eines oder mehrerer Alginate, wie z. B. Natriumalginat,
- ≥2 Gew.-% Hyaluronsäure und/oder
- ≥5 Gew.-% eines oder mehrerer weiterer natürlicher Polymere, wie z.B. Carrageen, sowie

≤ 10 Gew.-%, bevorzugt ≤5 Gew.-%, bevorzugter ≤1 Gew.-% Wasser, mit der Maßgabe, dass eine 1 gewichtsprozentige Lösung oder Suspension einer solchen gefriergetrockneten Zusammensetzung in Wasser bei 20 °C einen pH Wert zwischen pH 4,0 und pH 6,0 und einen Massenquellungsgrad von 20 bis 60 aufweist.

Eine weitere bevorzugte Ausführungsform enthält darüber hinaus ≥ 1,0 Gew.-% eines oder mehrerer Wirkstoffe, insbesondere Ascorbinsäure (Vitamin C) oder seine Derivate oder Salicylsäure oder seine Derivate wie Acetylsalicylsäure (ASS) oder Silberverbindungen wie Silbernitrat und andere in der Wundbehandlung gebräuchliche Wirkstoffe,
≤ 30 Gew.-% eines oder mehrerer Hilfsstoffe, wie insbesondere kosmetische Öle wie Neutralöl, Jojobaöl oder Squalan, mit der Maßgabe, dass eine 1 gewichtsprozentige Lösung oder Suspension einer solchen gefriergetrockneten Zusammensetzung in Wasser bei 20 °C einen pH Wert zwischen pH 4,0 und pH 6,0 und einen Massenquellungsgrad von 20 bis 100 aufweist.

Bevorzugt weist eine erfindungsgemäße gefriergetrocknete Zusammensetzung wie z.B. eine der vorstehend erwähnten Zusammensetzungen einen pH-Wert zwischen 4,0 und 6,0, bevorzugt zwischen pH 4,5 und pH 5,5 und besonders bevorzugt von pH 5,0 , gemessen in dessen 1 Gew.-% Lösung oder Suspension in Wasser bei 20 °C,

- eine Dichte von 0,005 $g/cm^3$ bis zu 0,8 $g/cm^3$ bevorzugt 0,01 $g/cm^3$ bis zu 0,8 $g/cm^3$,
- eine Fläche von 0,1 $cm^2$ bis 600 $cm^2$, bevorzugt 5 $cm^2$ bis 300 $cm^2$,
- eine Dicke von (kürzester Abstand zwischen zwei Punkten der Zusammensetzung) von ≥0,5 mm auf und/oder
- besitzt bevorzugt eine flächige Ausgestaltung, besonders bevorzugt die Form eines Sheets, eines Pads, einer Auflage oder einer Maske.

Die erfindungsgemäßen gefriergetrockneten Zusammensetzungen stellen poröse Zubereitungen mit homogener Verteilung der Inhaltsstoffe dar.

Die Auflösungs- bzw. Rehydratationsgeschwindigkeit der erfindungsgemäßen gefriergetrockneten Zusammensetzungen beträgt ≤ 20 Sekunden, noch bevorzugter ≤10 Sekunden. Die Messung erfolgt an erfindungsgemäßen gefriergetrockneten Zusammensetzungen mit einer Dicke von 1,1 - 1,5 mm, einer Fläche von 16 $cm^2$ und/oder einem Gewicht von 0,6 g. Vollständige Hydratation liegt dabei vor, wenn eine homogene Gelbildung ohne erkennbare inhomogene Bereiche mit einer Fläche > 0,1 $cm^2$ vorliegt.

Insbesondere zur Bestimmung der Benetzungsgeschwindigkeit wird die Zeit ermittelt, die eine definierte Materialprobe bis zur vollständigen und gleichmäßigen Durchfeuchtung mit einer definierten Menge Flüssigkeit benötigt. Dabei ist zu beachten, dass die Befeuchtungsflüssigkeit im Überschuss vorliegt, so dass eine vollständige Benetzung bzw. Durchfeuchtung möglich ist. Bevorzugt werden auch hierzu Materialproben von 4 x 4 cm Fläche (16 $cm^2$) und einer Schichtdicke von 1,1 - 1,5 mm eingesetzt und in 7,5 ml der Befeuchtungsflüssigkeit, wie z.B. Wasser oder in eine physiologische Flüssigkeit wie 0,9 %ige Kochsalzlösung, eingebracht und die Zeit bis zur vollständigen Benetzung bzw. bis zum vollständigen und gleichmäßigen Durchfeuchten gemessen. Eine vollständige Durchfeuchtung bzw. Benetzung kann in der Regel leicht anhand der Farbveränderung erkannt werden, da die befeuchteten Bereiche dunkler erscheinen als noch nicht befeuchtete Bereiche.

Die Benetzungsgeschwindigkeit der erfindungsgemäßen gefriergetrockneten Zusammensetzungen beträgt ebenfalls bevorzugt ≤ 20 Sekunden, bevorzugter ≤10 Sekunden, sowie noch bevorzugter ≤5 Sekunden, ganz besonders bevorzugt < 1 Sekunde.

Die Erfindung umfasst insbesondere die folgenden bevorzugten Ausführungsformen:

1. Gefriergetrocknete Zusammensetzung enthaltend

   a. mindestens ein Polymer auf Basis von Polyacrylsäuren und Salzen davon
   b. mindestens ein natürliches Polymer,
   c. gegebenenfalls mindestens ein weiteres von a) und b) verschiedenes Polymer sowie
   d. gegebenenfalls einen oder mehrere Wirk- und/oder Hilfsstoffe.

2. Gefriergetrocknete Zusammensetzung nach Ausführungsform 1, die eine optische Dichte ≥ 0,02 pro 1 mm Schichtdicke der gefriergetrockneten Zusammensetzung aufweist.

3. Gefriergetrocknete Zusammensetzung nach Ausführungsform 1 oder 2, deren 1 gewichtsprozentige Lösung oder Suspension in Wasser bei 20 °C einen pH-Wert von 3,0 bis 6,5 hat.

4. Gefriergetrocknete Zusammensetzung nach einer der Ausführungsformen 1 bis 3 die eine flächige Form, bevorzugt insbesondere die Form eines Sheets, einer Auflage oder einer Maske aufweist.

5. Gefriergetrocknete Zusammensetzung nach einer der Ausführungsformen 1 bis 4 mit einer Dicke von 0,5 bis 20 mm.

6. Gefriergetrocknete Zusammensetzung nach einer der Ausführungsformen 1 bis 5, die nicht chemisch vernetzt ist.

7. Gefriergetrocknete Zusammensetzung nach einer der Ausführungsformen 1 bis 6, die keine textilen Faserbestandteile enthält.

8. Gefriergetrocknete Zusammensetzung nach einer der Ausführungsformen 1 bis 7, die keine zusätzliche Trägerschicht aufweist.

9. Verfahren zur Herstellung einer gefriergetrockneten Zusammensetzung umfassend die Schritte

   a. Herstellen einer wässrigen Suspension oder einer Lösung mindestens eines natürlichen Polymers
   b. gegebenenfalls Einmischen mindestens eines weiteren Polymers, das von dem in Schritt a) oder e) verwendeten Polymer verschieden ist
   c. gegebenenfalls Einmischen eines oder mehrerer Wirk- und/oder Hilfsstoffe
   d. Einstellen des pH Werts der wässrigen Suspension oder Lösung auf einen pH-Wert von pH 3,0 bis pH 6,5
   e. Herstellen einer wässrigen Suspension oder einer Lösung eines Polymers auf Basis von Polyacrylsäuren und Salzen davon und Einstellen des pH Wertes der Lösung mit Laugen auf einen pH-Wert zwischen pH 3,0 und pH 6,5
   f. Vereinigung der unter a)-d) und der unter e) hergestellten Suspensionen oder Lösungen und gegebenenfalls Einstellung des pH-Wertes auf pH 3,0 bis 6,5
   g. Giessen oder Ausstreichen der Mischung in eine geeignete Form oder auf eine geeignete Fläche
   h. Gefrieren der Mischung und
   i. Gefriertrocknung der Mischung unter Bildung der gefriergetrockneten Zusammensetzung.

10. Verfahren nach Ausführungsform 9, worin in Schritt d), e) und f) der pH-Wert auf pH 4,5 bis 5,5 eingestellt wird.

11. Verfahren nach einer der Ausführungsformen 9 bis 10, worin das Einstellen des pH-Wertes durch Zugabe von anorganischen Laugen, vorzugsweise einer Kaliumhydroxid-Lösung erfolgt.

12. Verfahren nach einer der Ausführungsformen 9 bis 11, worin in Anschluss an Schritt i) die gefriergetrocknete Zusammensetzung durch Schneiden in eine gewünschte Form, vorzugsweise eine Sheet-, Auflagen- oder Masken-Form gebracht wird.

13. Verfahren nach Ausführungsform 12, worin die geschnittenen Zusammensetzungen eine Dicke von 0,5 bis 20 mm aufweisen.

14. Gefriergetrocknete Zusammensetzung erhältlich nach dem Verfahren nach einer der Ausführungsformen 9 bis 13.

15. Gefriergetrocknete Zusammensetzung nach einer der Ausführungsformen 1 bis 8 oder 14, worin mindestens ein Polymer auf Basis von Polyacrylsäuren und Salzen davon ausgewählt ist aus der Gruppe der Carbomere.

16. Gefriergetrocknete Zusammensetzung nach einer der Ausführungsformen 1 bis 8 oder 14 bis 15, worin mindestens ein natürliches Polymer ausgewählt ist aus der Gruppe der Polysaccharide, der Polyaminosaccharide und/oder der Glucosaminoglycane.

17. Gefriergetrocknete Zusammensetzung nach einer der Ausführungsformen 1 bis 8 oder 14 bis 16, worin mindestens ein natürliches Polymer ausgewählt ist aus der Gruppe der anionischen Hydrokolloide.

18. Gefriergetrocknete Zusammensetzung nach Ausführungsform 16 oder 17, worin mindestens ein natürliches Polymer ausgewählt ist aus der Gruppe der Alginate, bevorzugt aus der Gruppe der Natrium-Alginate.

19. Gefriergetrocknete Zusammensetzung nach einer der Ausführungsformen 1 bis 8 und 14 bis 18, worin mindestens ein weiteres natürliches Polymer ausgewählt aus der Gruppe des Carrageenans und seiner Derivate, der Hyaluronsäure und ihrer Derivate und/oder des Collagens und seiner Derivate.

20. Gefriergetrocknete Zusammensetzung nach einer der Ausführungsformen 1 bis 8 und 14 bis 19, worin mindestens ein natürliches Polymer ausgewählt ist aus der Gruppe der Alginate, bevorzugt aus der Gruppe der Natrium-Alginate und ein weiteres natürliches Polymer ausgewählt ist aus der Gruppe der Hyaluronsäure und ihrer Derivate.

21. Gefriergetrocknete Zusammensetzung nach einer der Ausführungsformen 1 bis 8 und 14 bis 20, worin mindestens ein natürliches Polymer ausgewählt ist aus der Gruppe der Alginate, bevorzugt aus der Gruppe der Natrium-Alginate, ein weiteres natürliches Polymer ausgewählt ist aus der Gruppe der Hyaluronsäure und ihrer Derivate und worin mindestens ein weiteres Polymer, ausgewählt aus der Gruppe der Carrageene enthalten ist.

22. Gefriergetrocknete Zusammensetzung nach einer der Ausführungsformen 1 bis 8 und 14 bis 21, worin mindestens ein weiteres Polymer, bevorzugt ausgewählt aus der Gruppe der modifizierten Cellulosen, bevorzugt Carboxymethylcellulose, besonders bevorzugt Natriumcarboxymethylcellulose enthalten ist.

23. Gefriergetrocknete Zusammensetzung nach einer der Ausführungsformen 1 bis 8 und 14 bis 22, worin mindestens ein Wirkstoff aus der Gruppe der kosmetischen Wirkstoffe wie vorzugsweise Ascorbinsäure oder Ihrer Derivate, vorzugsweise Ascorbyl-Glucosid oder Magnesium-Ascorbylphosphat enthalten ist

24. Gefriergetrocknete Zusammensetzung nach einer der Ausführungsformen 1 bis 8 und 14 bis 22, worin mindestens ein Wirkstoff aus der Gruppe der therapeutischen Wirkstoffe wie vorzugsweise solche zur Behandlung von Hautkrankheiten und/oder zur Wundbehandlung enthalten ist.

25. Gefriergetrocknete Zusammensetzung nach einer der Ausführungsformen 1 bis 8 und 14 bis 24, worin mindestens ein Hilfsstoff aus der Gruppe der kosmetischen Öle, bevorzugt Triglyceride, besonders bevorzugt Capryl/Capronsäure-Triglyceride und/oder Squalan und/oder Jojobaöl enthalten ist.

26. Gefriergetrocknete Zusammensetzung nach einer der Ausführungsformen 1 bis 8 und 14 bis 25, worin mindestens ein Hilfsstoff aus der Gruppe der Füllstoffe, besonders bevorzugt Mannitol enthalten ist.

27. Gefriergetrocknete Zusammensetzung nach einer der Ausführungsformen 1 bis 8 und 14 bis 26, die bei Zufuhr von hydrophilen Flüssigkeiten unter Bildung eines homogenen, feindispersen Gels rehydratisierbar ist, welches im Wesentlichen frei von makroskopischen Partikeln oder Faserbestandteilen mit einer Größe > 5 mm ist.

28. Gefriergetrocknete Zusammensetzung nach einer der Ausführungsformen 1 bis 8 und 14 bis 27, die eine Wasseraufnahmekapazität, dargestellt als Massenquellungsgrad von 20 bis 100 aufweist

29. Gefriergetrocknete Zusammensetzung nach einer der Ausführungsformen 1 bis 8 und 14 bis 28 die bei Zugabe von 96,5 g Wasser zu 3,5 g der gefriergetrockneten Zusammensetzung bei einer Temperatur von 15-25 °C und einem pH-Wert von 4,8 - 5,2 ein homogenes, feindisperses Gel mit einer Viskosität von 10 - 75 mPas bildet.

30. Verwendung der gefriergetrockneten Zusammensetzung nach einer der Ausführungsformen 1 bis 8 und 14 bis 29 als kosmetisches Mittel.

31. Verwendung nach Ausführungsform 30 als kosmetische Auflage, wie eine kosmetische Maske.

32. Verwendung der gefriergetrockneten Zusammensetzung nach einer der Ausführungsformen 1 bis 8 und 14 bis 29 als pharmazeutisches Mittel.

33. Verwendung der gefriergetrockneten Zusammensetzung nach einer der Ausführungsformen 1 bis 8 und 14 bis 29 als Wundauflage.

34. Verwendung einer gefriergetrockneten Zusammensetzung nach einer der Ausführungsformen 1 bis 8 und 14 bis 29 zur Herstellung eines pharmazeutischen Mittels zur Wundbehandlung.

35. Verwendung einer gefriergetrockneten Zusammensetzung nach einer der Ausführungsformen 1 bis 8 und 14 bis 29 zur Herstellung eines pharmazeutischen Mittels zur Behandlung chronischer Wunden, wie z.B. Ulcus cruris, Dekubitus.

36. Gefriergetrocknete Zusammensetzung nach einer der Ausführungsformen 1 bis 8 und 14 bis 29 zur Verwendung als Wundauflage.

37. Verwendung nach einer der Ausführungsformen 30 bis 35, worin die gefriergetrocknete Zusammensetzung auf die zu behandelnde Körperpartie aufgebracht wird, dort mit Wasser oder einer wässrigen Lösung eines oder mehrerer Wirkstoffe und/oder gegebenenfalls Hilfsstoffe angefeuchtet wird, durch mechanische Einwirkung und/oder Massieren vollständig zerfällt und verteilt wird

38. Verwendung nach einer der Ausführungsformen 33 bis 36, worin die gefriergetrocknete Zusammensetzung trocken auf die zu behandelnde Körperpartie oder auf die zu behandelnde Wunde aufgebracht wird, wo sie durch die vorhandenen Körperflüssigkeiten rehydratisiert und im Lauf der weiteren Behandlung entweder vollständigen resorbiert oder mit der Wundflüssigkeit oder anderen Wundbehandlungsflüssigkeiten ausgespült wird.

39. Kit-of-parts Kombination enthaltend mindestens eine gefriergetrocknete Zusammensetzung gemäß einer der Ausführungsformen 1 bis 8 und 14 bis 29 sowie mindestens eine wässrige Lösung, die einen oder mehrere Wirkstoffe und/oder gegebenenfalls einen oder mehrere Hilfsstoffe enthält, in zusammengehöriger, räumlicher Anordnung.

40. Verwendung der Kit-of-parts Kombination nach Ausführungsform 39 als kosmetisches Mittel.

41. Verwendung der Kit-of-parts Kombination nach Ausführungsform 39 als therapeutisches Mittel.

42. Verwendung nach einer der vorstehenden Ausführungsformen, die direkt durch den Endverbraucher erfolgt.

Die Erfindung wird durch die folgenden Beispiele näher veranschaulicht.

BEISPIELE

**Referenzbeispiel 1**

| | |
|---|---|
| 10,5g | Natriumcarboxymethylcellulose (CMC) |
| 10,5g | Natrium-Alginat |
| 7,5g | Carbomer |
| 1500ml | RO-Wasser |

a) Herstellung der Carbomer-Vormischung
7,5g Carbomer werden nach Herstellerangaben in 750 ml RO-Wasser aufgelöst.

b) Herstellung der Alginat/CMC-Vormischung
10,5g Carboxymethylcellulose und 1 0,5g Natrium-Aiginat werden in 750 ml RO-Wasser unter Rühren homogen gelöst, bis keine unaufgelösten Alginat/CMC-Partikel mehr sichtbar sind.

c) Herstellung des Gesamtansatzes
Die Carbomer-Vormischung aus Schritt a) wird durch Rühren mit der Alginat/CMC-Vormischung aus Schritt b) vereinigt bis eine homogene Mischung entsteht. Der pH-Wert der vereinigten Mischung wird mit verdünnter Kalilauge auf einen pH-Wert von 5,0-5,5 eingestellt.

d) Einfrieren
Der in c) hergestellte Alginat/CMC/Carbomer-Ansatz wird entweder durch Anblasen mit kalter Luft oder durch Aufbringen auf eine kalte Platte bei Temperaturen von < -20°C eingefroren.

e) Gefriertrocknen
Das Gefriertrocknen, des unter d) hergestellten gefrorenen Formköpers erfolgt nach dem Stand der Technik nach allgemein bekannten Gefriertrocknungsverfahren

f) Spalten und Konfektionieren
Die gefriergetrocknete Zusammensetzung wird in dünne Schichten mit einer Schichtdicke von 0,5 mm geschnitten. Die Schichten sind optisch dicht und das Material lässt sich in weniger als 10 Sekunden vollständig benetzen.

**Beispiel 2**

| | |
|---|---|
| 7,5g | Natriumcarboxymethylcellulose (CMC) |
| 4,5g | Carrageenan |
| 4,5g | Carbomer |
| 4,5g | Fischkollagenhydrolysat |
| 4,5g | Hyaluronsäure |
| 3,0g | Natrium-Alginat |
| 7,5g | Calcium-Alginat |
| 15,0g | Mannitol |
| 1500ml | RO-Wasser |

a) Herstellung der Carbomer-Vormischung
4,5g Carbomer werden nach Herstellerangaben in 500 ml RO-Wasser aufgelöst.

b) Herstellung der Alginat/CMC/Hyaluronsäure/Mannitol /Fischkollagenhydrolysat-Vormischung
Die restlichen Substanzen werden in 1000 ml RO-Wasser heiß gelöst, bis keine ungelösten Substanzen mehr erkennbar sind. Man lässt anschließend die Lösung auf Raumtemperatur erkalten.

c) Herstellung des Gesamtansatzes
Die Carbomer-Vormischung aus Schritt a) wird durch Rühren mit der Vormischung aus Schritt b) vereinigt bis eine homogene Mischung entsteht. Der pH-Wert der vereinigten Mischung wird mit verdünnter Kalilauge auf einen pH-Wert von 5,0-5,5 eingestellt.

d) Einfrieren
Der in c) hergestellte Ansatz wird entweder durch Anblasen mit kalter Luft oder durch Aufbringen auf eine kalte Platte bei Temperaturen von < -20°C eingefroren.

e) Gefriertrocknen
Das Gefriertrocknen, des unter d) hergestellten gefrorenen Formköpers erfolgt nach dem Stand der Technik nach allgemein bekannten Gefriertrocknungsverfahren .

f) Spalten und Konfektionieren
Die gefriergetrocknete Zusammensetzung wird in dünne Schichten mit einer Schichtdicke von 1,5 mm geschnitten. Die Schichten sind optisch dicht und das Material lässt sich in weniger als 10 Sekunden vollständig benetzen.

**Beispiel 3**

| | |
|---|---|
| 7,5g | Natrium-Alginat |
| 4,5g | Carrageenan |
| 11,25g | Carbomer |
| 1500ml | RO-Wasser |

a) Herstellung der Carbomer-Vormischung
11,25g Carbomer werden nach Herstellerangaben in 750 ml RO-Wasser aufgelöst.

b) Herstellung der Alginat/Carrageenan-Vormischung
7,5g Na-Alginat und 4,5g Carrageenan werden in 750 ml heißem RO-Wasser unter Rühren homogen gelöst, bis keine unaufgelösten Partikel mehr sichtbar sind.

c) Herstellung des Gesamtansatzes
Die Carbomer-Vormischung aus Schritt a) wird durch Rühren mit der Alginat/Carrageenan-Vormischung aus Schritt b) vereinigt bis eine homogene Mischung entsteht. Der pH-Wert der vereinigten Mischung wird mit verdünnter Kalilauge auf einen pH-Wert von 5,0 eingestellt.

d) Einfrieren
Der in c) hergestellte Alginat/Carrageenan/Carbomer-Ansatz wird entweder durch anblasen mit kalter Luft oder durch aufbringen auf eine kalte Platte bei Temperaturen von < -20°C eingefroren.

e) Gefriertrocknen
Das Gefriertrocknen, des unter d) hergestellten gefrorenen Formköpers erfolgt nach dem Stand der Techniknach allgemein bekannten Gefriertrocknungsverfahren

f) Spalten und Konfektionieren
Die gefriergetrocknete Zusammensetzung wird in dünne Schichten mit einer Schichtdicke von 2,0 mm geschnitten. Die Schichten sind optisch dicht und das Material lässt sich in > 30 Sekunden vollständig benetzen.

**Beispiel 4**

| | |
|---|---|
| 10,5g | Natriumcarboxymethylcellulose (CMC) |
| 10,5g | Natrium-Alginat |

(fortgesetzt)

| | |
|---|---|
| 7,5g | Carbomer |
| 1,5g | Hyaluronsäure |
| 1500ml | RO-Wasser |

a) Herstellung der Carbomer-Vormischung
7,5g Carbomer werden nach Herstellerangaben in 750 ml RO-Wasser aufgelöst.

b) Herstellung der Alginat/Hyaluronsäure/CMC-Vormischung
10,5g Carboxymethylcellulose, 1,5g Hyaluronsäure und 10,5g Natrium-Alginat werden in 750 ml RO-Wasser unter Rühren homogen gelöst, bis keine unaufgelösten Alginat/Hyaluronsäure/CMC-Partikel mehr sichtbar sind.

c) Herstellung des Gesamtansatzes
Die Carbomer-Vormischung aus Schritt a) wird durch Rühren mit der Alginat/Hyaluronsäure/CMC-Vormischung aus Schritt b) vereinigt bis eine homogene Mischung entsteht. Der pH-Wert der vereinigten Mischung wird mit verdünnter Kalilauge auf einen pH-Wert von 5,0-5,5 eingestellt.

d) Einfrieren
Der in c) hergestellte Alginat/Hyaluronsäure/CMC/Carbomer-Ansatz wird entweder durch Anblasen mit kalter Luft oder durch Aufbringen auf eine kalte Platte bei Temperaturen von < -20°C eingefroren.

e) Gefriertrocknen
Das Gefriertrocknen, des unter d) hergestellten gefrorenen Formköpers erfolgt nach dem Stand der Technik nach allgemein bekannten Gefriertrocknungsverfahren

f) Spalten und Konfektionieren
Die gefriergetrocknete Zusammensetzung wird in dünne Schichten mit einer Schichtdicke von 0,5 mm geschnitten. Die Schichten sind optisch dicht und das Material lässt sich in weniger als 5 Sekunden vollständig benetzen.

**Beispiel 5**

| | |
|---|---|
| 3,0g | Natriumcarboxymethylcellulose (CMC) |
| 4,5g | Carrageenan |
| 4,5g | Carbomer |
| 4,5g | Fischkollagenhydrolysat |
| 1,5g | Hyaluronsäure |
| 7,5g | Natrium-Alginat |
| 1500ml | RO-Wasser |

a) Herstellung der Carbomer-Vormischung
4,5g Carbomer werden nach Herstellerangaben in 500 ml RO-Wasser aufgelöst.

b) Herstellung der Alginat / CMC / Carrageen / Hyaluronsäure / Fischkollagenhydrolysat-Vormischung
Die restlichen Substanzen werden in 1000 ml RO-Wasser heiß gelöst, bis keine ungelösten Substanzen mehr erkennbar sind. Man lässt anschließend die Lösung auf Raumtemperatur erkalten.

c) Herstellung des Gesamtansatzes
Die Carbomer-Vormischung aus Schritt a) wird durch Rühren mit der Vormischung aus Schritt b) vereinigt bis eine homogene Mischung entsteht. Der pH-Wert der vereinigten Mischung wird mit verdünnter Kalilauge auf einen pH-Wert von 5,0-5,5 eingestellt.

d) Einfrieren
Der in c) hergestellte Ansatz wird entweder durch Anblasen mit kalter Luft oder durch Aufbringen auf eine kalte

Platte bei Temperaturen von < -20°C eingefroren.

e) Gefriertrocknen
Das Gefriertrocknen, des unter d) hergestellten gefrorenen Formköpers erfolgt nach dem Stand der Technik nach allgemein bekannten Gefriertrocknungsverfahren.

f) Spalten und Konfektionieren
Die gefriergetrocknete Zusammensetzung wird in dünne Schichten mit einer Schichtdicke von 1,5 mm geschnitten. Die Schichten sind optisch dicht und das Material lässt sich in weniger als 1 Sekunde vollständig benetzen.

**Beispiel 6**

| | |
|---|---|
| 11,25g | Natrium-Alginat |
| 4,5g | Carrageenan |
| 11,25g | Carbomer |
| 1,5g | Hyaluronsäure |
| 7,5g | Natriumcarboxymethylcellulose(CMC) |
| 7,5g | Mannitol |
| 1500ml | RO-Wasser |

a) Herstellung der Carbomer-Vormischung
11,25g Carbomer werden nach Herstellerangaben in 750 ml RO-Wasser aufgelöst.

b) Herstellung der Alginat / Hyaluronsäure / Carrageenan / CMC / Mannitol-Vormischung
Die restlichen Substanzen werden in 750 ml heißem RO-Wasser gelöst, bis keine ungelösten Partikel mehr sichtbar sind. Man lässt anschließend die Lösung auf Raumtemperatur erkalten.

c) Herstellung des Gesamtansatzes
Die Carbomer-Vormischung aus Schritt a) wird durch Rühren mit der Alginat / Hyaluronsäure / Carrageenan / CMC / Mannitol-Vormischung aus Schritt b) vereinigt bis eine homogene Mischung entsteht. Der pH-Wert der vereinigten Mischung wird mit verdünnter Kalilauge auf einen pH-Wert von 5,0 eingestellt.

d) Einfrieren
Der in c) hergestellte Ansatz wird entweder durch anblasen mit kalter Luft oder durch aufbringen auf eine kalte Platte bei Temperaturen von < -20°C eingefroren.

e) Gefriertrocknen
Das Gefriertrocknen, des unter d) hergestellten gefrorenen Formköpers erfolgt nach dem Stand der Technik nach allgemein bekannten Gefriertrocknungsverfahren

f) Spalten und Konfektionieren
Die gefriergetrocknete Zusammensetzung wird in dünne Schichten mit einer Schichtdicke von 2,0 mm geschnitten. Die Schichten sind optisch dicht und das Material lässt sich in weniger als 1 Sekunde vollständig benetzen.

**Beispiel 7**

| | |
|---|---|
| 7,5g | Natriumcarboxymethylcellulose (CMC) |
| 4,5g | Carrageenan |
| 11,25g | Carbomer |
| 1,5g | Hyaluronsäure |
| 11,25g | Natrium-Alginat |
| 7,5g | Mannitol |

(fortgesetzt)

| | |
|---|---|
| 1,5g | Jojobaöl |
| 0,75g | Magnesiumascorbylphosphat |
| 1500ml | RO-Wasser |

a) Herstellung der Carbomer-Vormischung
11,25g Carbomer werden nach Herstellerangaben in 700 ml RO-Wasser aufgelöst.

b) Herstellung der Alginat / Hyaluronsäure / Carrageenan / CMC / Mannitol / Jojobaöl-Vormischung
Die restlichen Substanzen, außer das Jojojobaöl und das Magnesiumascorbylphosphat, werden in 750 ml heißem RO-Wasser gelöst, bis keine ungelösten Partikel mehr sichtbar sind. Man lässt anschließend die Lösung auf Raumtemperatur erkalten. Dann wird das Jojobaöl untergemischt.

c) Herstellung der Magnesiumascorbylphosphat-Vormischung
Die 0,75g Magnesiumascorbylphosphat werden in 50 ml RO-Wasser gelöst, bis keine ungelösten Partikel mehr sichtbar sind.

d) Herstellung des Gesamtansatzes
Die Carbomer-Vormischung aus Schritt a) wird durch Rühren mit der Alginat / Hyaluronsäure / Carrageenan / CMC / Mannitol Jojobaöl-Vormischung aus Schritt b) vereinigt bis eine homogene Mischung entsteht. Die Wirkstofflösung aus Schritt c) wird ebenfalls durch Rühren homogen untergemischt. Dann wird der pH-Wert der vereinigten Mischung mit verdünnter Kalilauge auf einen pH-Wert von 5,0 eingestellt.

e) Einfrieren
Der in d) hergestellte Ansatz wird entweder durch anblasen mit kalter Luft oder durch aufbringen auf eine kalte Platte bei Temperaturen von < -20°C eingefroren.

f) Gefriertrocknen
Das Gefriertrocknen, des unter e) hergestellten gefrorenen Formköpers erfolgt nach dem Stand der Technik nach allgemein bekannten Gefriertrocknungsverfahren

g) Spalten und Konfektionieren
Die gefriergetrocknete Zusammensetzung wird in dünne Schichten mit einer Schichtdicke von 2,0 mm geschnitten. Die Schichten sind optisch dicht und das Material lässt sich in weniger als 1 Sekunde vollständig benetzen.

**Beispiel 8**

Der Einfluss der strukturbildenden Polymere und deren verschiedener Kombinationsmöglichkeiten in gefriergetrockneten Sheet-förmigen Zusammensetzungen im Sinne der vorliegenden Erfindung wurde anhand der Benetzungsgeschwindigkeit sowie des Flüssigkeitshalte vermögen untersucht.

Gegenstand der Untersuchungen waren dabei Zusammensetzungen, die lediglich eines der strukturbildenden Polymere Carbomer, Alginat, Hyaluronsäure oder Carrageen enthalten, Zusammensetzungen mit einer Kombination von zwei dieser strukturbildenden Polymere, wie sie z.B. auch aus dem Stand der Technik bekannt sind, sowie die erfindungsgemäß bevorzugten Zusammensetzungen, die eine Kombination der drei strukturbildenden Polymere Carbomer, Alginat und Hyaluronsäure enthalten. Außerdem wurde eine weitere bevorzugte Zusammensetzung, die neben diesen drei Polymeren außerdem Carrageen enthält, untersucht.

Dazu wurde, soweit herstellungsbedingt möglich, die Benetzungsgeschwindigkeit sowie das Flüssigkeitshaltevermögen mit dem Massenquellungsindex $Q_M$ sowie dem gehaltenen Flüssigkeitsvolumen pro Gramm Zusammensetzung bestimmt. Die Versuchsdurchführung erfolgte gemäß der vorstehend beschriebenen Methoden unter den dort angegebenen Versuchsbedingungen.

Alle Versuche wurden sowohl mit Wasser als auch mit einer physiologischen Kochsalzlösung (0,9 %ige NaCl-Lösung) durchgeführt.

Die Ergebnisse sind in den nachfolgenden Tabellen zusammengefasst, mit den folgenden Bedeutungen für die Anmerkungen:

* bei hohem TS (Trockensubstanz) zu viskos bzw. Gelieren bei < 40 °C / nicht pumpbar, bei niedrigem TS instabil

im gefrorenen Zustand

** mechanisch nicht schneidstabil; dies kann sowohl bedeuten, dass die Struktur der Matrix zu offenporig ist und beim Schneiden zerbröckelt, oder dass von vorneherein keine zusammenhängende schneidfähige Matrix vorhanden ist, sondern lediglich locker aneinandergelagerte Fasern, die beim Schneiden vom Messer zerdrückt und nicht geschnitten werden

*** teuer

Benetzungsgeschwindigkeit:

Zusammensetzungen mit einem strukturbildenden Polymer

| Beispiel | Polymer | Verarbeitbarkeit | Benetzungsgeschwindigkeit in Wasser | Benetzungsgeschwindigkeit in 0,9 %iger NaCl-Lsg. |
|---|---|---|---|---|
| | Carbomer | nicht herstellbar (*/**) | nicht messbar | nicht messbar |
| | Alginat | nicht herstelibar (**) | nicht messbar | nicht messbar |
| | Hyaluronsäure | nicht herstellbar (**/***) | nicht messbar | nicht messbar |
| | Carrageenan | nicht herstellbar (*/**) | nicht messbar | nicht messbar |

Zusammensetzungen mit zwei strukturbildenden Polymeren

| Beispiel | Polymere | Verarbeitbarkeit | Benetzungsgeschwindigkeit in Wasser | Benetzungsgeschwindigkeit in 0,9 %iger NaCl-Lsg. |
|---|---|---|---|---|
| Stand d. Technik | Carbomer + Alginat | schlecht (**) | Zerfall in kleine Stücke | Zerfall in kleine Stücke |
| Stand d. Technik | Carbomer + Hyaluronsäure | nicht herstellbar (*/**/***) | nicht messbar | nicht messbar |
| | Carbomer + Carrageenan | nicht herstellbar (*) | nicht messbar | nicht messbar |
| | Alginat + Hyaluronsäure | nicht herstellbar (**) | nicht messbar | nicht messbar |
| | Alginat + Carrageenan | nicht herstellbar (**) | nicht messbar | nicht messbar |
| | Hyaluronsäure + Carrageenan | nicht herstellbar (*/***) | nicht messbar | nicht messbar |

Zusammensetzungen mit mindestens drei strukturbildenden Polymeren

| Beispiel | Polymere | Verarbeitbarkeit | Benetzungsgeschwindigkeit in Wasser | Benetzungsgeschwindigkeit in 0,9 %iger NaCl-Lsg. |
|---|---|---|---|---|
| 4 | Carbomer + Alginat + Hyaluronsäure | gut | < 5 Sekunden | < 5 Sekunden |
| 3 | Carbomer + Alginat + Carrageenan | gut | > 30 Sekunden | > 30 Sekunden |
| Referenzbeispiel | Carbomer + Hyaluronsäure + Carrageenan | gut | > 10 Sekunden | > 10 Sekunden |
| Referenzbeispiel | Alginat + Hyaluronsäure + Carrageenan | gut | > 60 Sekunden | > 60 Sekunden |
| 5, 6, 7 | Carbomer + Alginat + Hyaluronsäure + Carrageenan | gut | ≤ 1 Sekunde | ≤ 1 Sekunde |

Mit Zusammensetzungen, die mindestens drei strukturbildende Polymere enthalten, wurden Versuche zur Flüssigkeitsaufnahme oder -haltekapazität durchgeführt, deren Ergebnisse wie folgt dargestellt sind:

| Beispiel | Polymere | ∅ Wasser ml/g | ∅ 0,9%ige NaCl-Lsgml/g | ∅ $Q_m$ in Wasser | ∅ 0,9%ige NaCl-Lsgml/g | ∅ $\Delta Q_m$ |
|---|---|---|---|---|---|---|
| 4 | Carbomer + Alginat + Hyaluronsäure | 35 | 33 | 36 | 34 | 2 |
| 3 | Carbomer + Alginat + Carrageenan | 48 | 30 | 49 | 31 | 18 |
| Referenzbeispiel | Carbomer + Hyaluronsäure + Carrageenan | 61 | 28 | 62 | 29 | 33 |
| Referenzbeispiel | Alginat + Hyaluronsäure + Carrageenan | 23 | 19 | 24 | 20 | 4 |
| 5, 6, 7 | Carbomer + Alginat + Hyaluronsäure + Carrageenan | 35 | 28 | 36 | 29 | 7 |

Bei der Herstellung der verschiedenen Matrices zeigte sich, dass Zusammensetzungen, die lediglich ein Polymer enthielten, entweder im flüssigen / viskosen Zustand nicht verarbeitbar waren oder im gefriergetrockneten Zustand keine ausreichende mechanische Stabilität zur Herstellung von geschnittenen Sheets oder Pads aufweisen konnten.

Aus der Gruppe der Zusammensetzungen mit einer Kombination von zwei Polymeren zeigten sich ähnliche Probleme bei der Herstellung. Hier konnten zwar sheet-förmige Zusammensetzungen mit einer Carbomer/Alginat-Kombination erhalten werden, diese zeigten jedoch in der Applikation bzw. beim Kontakt mit Flüssigkeit ebenfalls eine ausgesprochen schlechte mechanische Stabilität, indem sie in kleine Stücke zerfielen.

Lediglich aus Zusammensetzungen mit einer Kombination von mindestens drei der strukturbildenden Polymere konnten mechanisch stabile, Sheet-förmige Materialien erhalten werden.

Hinsichtlich der Benetzungs- und Flüssigkeitshalteeigenschaften, dieser Materialien zeigten sich deutliche Unterschiede.

Ausschließlich Zusammensetzungen, die eine Kombination von Carbomer, Alginat und Hyaluronsäure enthalten, weisen ausreichend hohe Benetzungsgeschwindigkeiten auf. Für die übrigen Kombinationsmöglichkeiten konnten zwar nun mechanisch stabile Zusammensetzungen erhalten werden, diese zeigten jedoch deutlich nachteilige Benetzungsgeschwindigkeiten von >10 bis >60 Sekunden und damit ein extrem schlechtes Flüssigkeitsaufnahmeverhalten.

Weiterhin zeigte sich, dass das ausgesprochen gute Benetzungs- und das Flüssigkeitsaufnahmeverhalten von Zusammensetzungen mit Carbomer, Alginat und Hyaluronsäure durch die Zugabe von Carrageen noch weiter verbessert werden kann.
Dabei zeigen die Versuche jedoch auch, dass die Art des verwendeten Alginats einen Einfluss auf die Benetzungseigenschaften hat. Ein Vergleich von Beispiel 2, worin Carbomer und Hyaluronsäure mit Natrium- und Calcium-Alginat kombiniert wurden, mit Beispiel 5, worin eine vergleichbare Zusammensetzung ausschließlich mit Natrium-Alginat gezeigt ist, macht diesen Einfluss deutlich. Ca-Ionen haben bekanntermaßen Alginat-vernetzende Eigenschaften, was sich in der Benetzung bzw. Auflösung der Zusammensetzungen nachteilig niederschlägt. So weisen Zusammensetzungen nach Beispiel 2 mit < 10 Sekunden höhere Benetzungsgeschwindigkeiten auf, als solche nach Beispiel 5, die kein Ca-Alginat enthalten, und eine Benetzungsgeschwindigkeit von < 1 Sekunde aufweisen.

Daraus ergibt sich somit, dass lediglich Zusammensetzungen die eine Kombination von Carbomer, Alginat, insbesondere bevorzugt von Natrium-Alginat, und Hyaluronsäure enthalten, die erfindungsgemäß bevorzugten Benetzungsgeschwindigkeiten von < 10, bevorzugter von < 5, noch bevorzugter < 1 Sekunden erreichen.

Die Ergebnisse zeigen weiterhin, dass Zusammensetzungen, die eine Kombination aus Carbomer, Alginat und Hyaluronsäure enthalten, eine bessere Kapazität zur Speicherung von physiologischen Flüssigkeiten besitzen. Insbesondere zeigte sich dabei, dass die Flüssigkeitshaltekapazität von Zusammensetzungen mit Carbomer, Alginat und Hyaluronsäure im Vergleich der Speicherung von reinem Wasser zur Speicherung von physiologischen Flüssigkeiten gleichbleibend gut war bzw. stabil gehalten werden konnte, was insbesondere aus der deutlich geringeren Abnahme des Massenquellungsindexes ($\Delta Q_M$) beim Vergleich dieser Werte deutlich wird. Eine ähnlich stabile Flüssigkeitshaltekapazität wiesen nur Zusammensetzungen aus Alginat, Hyaluronsäure und Carrageen (ohne Carbomer) auf, wobei solche Zusammensetzungen neben einer extrem schlechten Benetzungsgeschwindigkeit auch die schlechteste absolute Flüssigkeitshaltekapazität zeigten.

Zusammenfassend lässt sich festhalten, dass unter den untersuchten Polymerkombinationen lediglich die Zusammensetzungen, die eine Kombination von Carbomer, Alginat und Hyaluronsäure enthalten, die erfindungsgemäß gewünschte Kombination von sehr guter Flüssigkeitsaufnahme bzw. Benetzungsgeschwindigkeit von < 10 Sekunden und hoher, auch in Elektrolyt-haltigen Flüssigkeiten stabiler, Flüssigkeitshaltekapazität aufweisen.

## Patentansprüche

1. Gefriergetrocknete Zusammensetzung enthaltend

   a) mindestens ein Polymer auf Basis von Polyacrylsäuren und Salzen davon
   b) mindestens ein natürliches Polymer ausgewählt aus der Gruppe der Alginate,
   c) mindestens ein weiteres natürliches Polymer ausgewählt aus der Gruppe des Carrageenans und seiner Derivate, der Hyaluronsäure und ihrer Derivate und/oder des Collagens und seiner Derivate,
   d) gegebenenfalls mindestens ein weiteres von a), b) und c) verschiedenes Polymer sowie
   e) gegebenenfalls einen oder mehrere Wirk- und/oder Hilfsstoffe.

2. Gefriergetrocknete Zusammensetzung nach Anspruch 1, die eine optische Dichte ≥ 0,02 pro 1 mm Schichtdicke der gefriergetrockneten Zusammensetzung aufweist.

3. Gefriergetrocknete Zusammensetzung nach Anspruch 1 oder 2, deren 1 gewichtsprozentige Lösung oder Suspension in Wasser bei 20 °C einen pH-Wert von 3,0 bis 6,5 hat.

4. Gefriergetrocknete Zusammensetzung nach einem der Ansprüche 1 bis 3, die nicht chemisch vernetzt ist und/oder die keine textilen Faserbestandteile enthält

5. Verfahren zur Herstellung einer gefriergetrockneten Zusammensetzung umfassend die Schritte

   a) Herstellen einer wässrigen Suspension oder einer Lösung mindestens eines natürlichen Polymers ausgewählt aus der Gruppe der Alginate und mindestens eines weiteren natürlichen Polymers ausgewählt aus der Gruppe des Carrageenans und seiner Derivate, der Hyaluronsäure und ihrer Derivate und/oder des Collagens und seiner Derivate
   b) gegebenenfalls Einmischen mindestens eines weiteren Polymers, das von den in Schritt a) oder e) verwendeten Polymeren verschieden ist
   c) gegebenenfalls Einmischen eines oder mehrerer Wirk- und/oder Hilfsstoffe
   d) Einstellen des pH Werts der wässrigen Suspension oder Lösung auf einen pH-Wert von pH 3,0 bis pH 6,5
   e) Herstellen einer wässrigen Suspension oder einer Lösung eines Polymers auf Basis von Polyacrylsäuren und Salzen davon und Einstellen des pH Wertes der Lösung mit Laugen/Säuren, bevorzugt Laugen auf einen pH-Wert zwischen pH 3,0 und pH 6,5
   f) Vereinigung der unter a)-d) und der unter e) hergestellten Suspensionen oder Lösungen und gegebenenfalls Einstellung des pH-Wertes auf pH 3,0 bis 6,5
   g) Giessen oder Ausstreichen der Mischung in eine geeignete Form oder auf eine geeignete Fläche
   h) Gefrieren der Mischung und
   i) Gefriertrocknung der Mischung unter Bildung der gefriergetrockneten Zusammensetzung.

6. Verfahren nach Anspruch 5, worin in Anschluss an Schritt i) die gefriergetrocknete Zusammensetzung durch Schneiden in eine gewünschte Form, insbesondere eine Sheet-, Auflagen- oder Masken-Form gebracht wird.

7. Gefriergetrocknete Zusammensetzung erhältlich nach dem Verfahren nach einem der Ansprüche 5 bis 6.

8. Gefriergetrocknete Zusammensetzung nach einem der Ansprüche 1 bis 4 und 7, worin mindestens ein Polymer auf Basis von Polyacrylsäuren und Salzen davon ausgewählt ist aus der Gruppe der Carbomere.

9. Gefriergetrocknete Zusammensetzung nach einem der Ansprüche 1 bis 4 und 7 bis 8, worin mindestens ein weiteres natürliches Polymer ausgewählt ist aus der Gruppe der Hyaluronsäure und ihrer Derivate.

10. Gefriergetrocknete Zusammensetzung nach einem der Ansprüche 1 bis 4 und 7 bis 8, worin mindestens ein weiteres natürliches Polymer ausgewählt ist aus der Gruppe der Hyaluronsäure und ihrer Derivate und mindestens ein

weiteres natürliches Polymer ausgewählt ist aus der Gruppe des Carrageenans und seiner Derivate.

**11.** Gefriergetrocknete Zusammensetzung nach einem der Ansprüche 1 bis 4 und 7 bis 11, worin mindestens ein Hilfsstoff aus der Gruppe der kosmetischen Öle, bevorzugt Triglyceride, besonders bevorzugt Capryl/Capronsäure-Triglyceride enthalten ist.

**12.** Gefriergetrocknete Zusammensetzung nach einem der Ansprüche 1 bis 4 und 7 bis 11 zur Verwendung als pharmazeutisches Mittel.

**13.** Gefriergetrocknete Zusammensetzung nach einem der Ansprüche 1 bis 4 und 7 bis 11 zur Verwendung als Wundauflage.

**14.** Verwendung der gefriergetrockneten Zusammensetzung nach einem der Ansprüche 1 bis 4 und 7 bis 11 als kosmetisches Mittel.

**15.** Kit-of-parts Kombination enthaltend mindestens eine gefriergetrocknete Zusammensetzung gemäß einem der Ansprüche 1 bis 4 und 7 bis 13 sowie mindestens eine wässrige Lösung, die einen oder mehrere Wirkstoffe und/oder gegebenenfalls einen oder mehrere Hilfsstoffe enthält, in zusammengehöriger, räumlicher Anordnung.

**Claims**

**1.** Freeze-dried composition containing

a) at least one polymer based on polyacrylic acids and salts thereof
b) at least one natural polymer selected from the group of alginates,
c) at least one further natural polymer selected from the group of the carrageenan and its derivatives, the hyaluronic acid and its derivatives and / or the collagen and its derivatives,
d) optionally at least one further of a), b) and c) different polymer as well
e) optionally one or more active substance and/or excipients.

**2.** The freeze-dried composition of claim 1 having an optical density ≥ 0.02 per 1 mm layer thickness of the freeze-dried composition.

**3.** Freeze-dried composition according to claim 1 or 2, whose 1 percent by weight solution or suspension in water at 20 °C has a pH of 3.0 to 6.5.

**4.** Freeze-dried composition according to one of claims 1 to 3, which is not chemically crosslinked and / or contains no textile fiber constituents.

**5.** A process for preparing a freeze-dried composition comprising the steps

a) preparing an aqueous suspension or a solution of at least one natural polymer selected from the group of alginates and at least one other natural polymer selected from the group of carrageenans and its derivatives, hyaluronic acid and its derivatives and / or collagen and its derivatives
b) optionally mixing in at least one further polymer which is different from the polymers used in step a) or e)
c) optionally mixing in one or more active ingredients and / or adjuvants
d) adjusting the pH of the aqueous suspension or solution to a pH of from pH 3.0 to pH 6.5
e) preparation of an aqueous suspension or a solution of a polymer based on polyacrylic acids and salts thereof and adjusting the pH of the solution with alkalis / acids, preferably alkalis to a pH value between pH 3.0 and pH 6.5
f) combining the suspensions or solutions prepared under a) -d) and under e) and optionally adjusting the pH to pH 3.0 to 6.5
g) pouring or spreading the mixture into a suitable mold or surface
h) freezing the mixture and
i) freeze-drying the mixture to form the freeze-dried composition.

**6.** The method according to claim 5, wherein subsequent to step i) the freeze-dried composition is brought by cutting into a desired shape, in particular a sheet, edition or mask form.

7. A freeze-dried composition obtainable by the process according to any one of claims 5 to 6.

8. A freeze-dried composition according to any one of claims 1 to 4 and 7, wherein at least one polymer based on polyacrylic acids and salts thereof is selected from the group of carbomers.

9. The freeze-dried composition according to any one of claims 1 to 4 and 7 to 8, wherein at least one other natural polymer is selected from the group of hyaluronic acid and its derivatives.

10. The freeze-dried composition according to any one of claims 1 to 4 and 7 to 8, wherein at least one other natural polymer is selected from the group of hyaluronic acid and its derivatives and at least one other natural polymer is selected from the group of carrageenans and its derivatives.

11. Freeze-dried composition according to any one of claims 1 to 4 and 7 to 11, wherein at least one excipient from the group of cosmetic oils, preferably triglycerides, particularly preferably Capryi / caproic acid triglycerides is included.

12. A freeze-dried composition according to any one of claims 1 to 4 and 7 to 11 for use as a pharmaceutical agent.

13. A freeze-dried composition according to any one of claims 1 to 4 and 7 to 11 for use as a wound dressing.

14. Use of the freeze-dried composition according to any one of claims 1 to 4 and 7 to 11 as a cosmetic agent.

15. Kit-of-parts combination containing at least one freeze-dried composition according to any one of claims 1 to 4 and 7 to 13 and at least one aqueous solution containing one or more active ingredients and / or optionally one or more excipients, in a co-located, spatial arrangement,

**Revendications**

1. Une composition lyophilisée contenant

a) au moins un polymère à base d'acides polyacryliques et de leurs sels
b) au moins un polymère naturel choisi dans le groupe des alginates,
c) au moins un autre polymère naturel choisi dans le groupe de la carraghénine et ses dérivés, l'acide hyaluronique et ses dérivés et / ou de collagène et ses dérivés,
d) éventuellement au moins un autre de a), b) et c) polymère différent ainsi
e) optionnellement un ou plusieurs substance active et / ou excipients.

2. Composition lyophilisée selon la revendication 1, qui a une densité optique ≥ épaisseur de couche 0,02 mm par 1 de la composition lyophilisée.

3. Composition lyophilisée selon la revendication 1 ou 2, la solution ou suspension à 1 pour cent en poids a un pH de 3,0 à 6,5 dans l'eau à 20 ° C

4. Composition lyophilisée selon l'une quelconque des revendications 1 à 3, qui est non réticulé chimiquement et / ou ne contient pas de constituants de fibres textiles.

5. Un Procédé de préparation d'une composition lyophilisée comprenant les étapes suivantes :

a) préparation d'une suspension aqueuse ou une solution d'au moins un polymère naturel choisi dans le groupe des alginates et au moins un autre polymère naturel choisi dans le groupe de la carraghénine et ses dérivés, l'acide hyaluronique et ses dérivés et / ou de collagène et ses dérivés
b) éventuellement, mélanger au moins un autre polymère qui est différent de ceux des polymères utilisés dans l'étape a) ou e)
c) éventuellement mélanger dans un ou plusieurs substance active et / ou excipients.
d) ajuster le pH de la suspension ou solution aqueuse à une valeur de pH allant de pH 3,0 à pH 6,5
e) la préparation d'une suspension aqueuse ou une solution d'un polymère à base d'acides polyacryliques et leurs sels et en ajustant la valeur du pH de la solution avec les bases / acides, de préférence des alcalis à une valeur de pH entre pH 3,0 et pH 6,5

f) l'union des suspensions ou solutions préparées sous a) -d) et e) et, le cas échéant, ajuster le pH à un pH de 3,0 à 6,5
g) verser ou répandre le mélange dans un moule ou une surface appropriée
h) congeler le mélange et
i) lyophiliser le mélange pour former la composition lyophilisée.

**6.** Procédé selon la revendication 5, dans lequel, après l'étape i), la composition lyophilisée est amenée par découpage en une forme désirée, en particulier une feuille, une édition ou un masque.

**7.** Composition lyophilisée pouvant être obtenue par le procédé selon l'une quelconque des revendications 5 à 6.

**8.** Composition lyophilisée selon l'une quelconque des revendications 1 à 4 et 7, dans laquelle au moins un polymère à base d'acides polyacryliques et de ses sels est choisi dans le groupe des carbomères.

**9.** Composition lyophilisée selon l'une quelconque des revendications 1 à 4 et 7 à 8, dans laquelle au moins un autre polymère naturel est choisi dans le groupe de l'acide hyaluronique et de ses dérivés.

**10.** Composition lyophilisée selon l'une quelconque des revendications 1 à 4 et 7 à 8, dans laquelle au moins un autre polymère naturel est choisi dans le groupe de l'acide hyaluronique et ses dérivés et au moins un autre polymère naturel est choisi dans le groupe des carraghénanes et ses dérivés.

**11.** Composition lyophilisée selon l'une quelconque des revendications 1 à 4 et 7 à 11, dans laquelle au moins un excipient du groupe des huiles cosmétiques, de préférence des triglycérides, de manière particulièrement préférée des triglycérides Capryi / acide caproïque est inclus.

**12.** Composition lyophilisée selon l'une quelconque des revendications 1 à 4 et 7 à 11 destinée à être utilisée comme agent pharmaceutique.

**13.** Composition lyophilisée selon l'une quelconque des revendications 1 à 4 et 7 à 11, destinée à être utilisée comme pansement pour plaie.

**14.** Utilisation de la composition lyophilisée selon l'une quelconque des revendications 1 à 4 et 7 à 11 comme agent cosmétique.

**15.** Combinaison de kit-de-pièces contenant au moins une composition lyophilisée selon l'une quelconque des revendications 1 à 4 et 7 à 13 et au moins une solution aqueuse contenant un ou plusieurs principes actifs et / ou éventuellement un ou plusieurs excipients, dans un arrangement spatial co-localisé,

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10195737
- WO 0022083 A
- WO 05123034 A
- GB 2431104 A
- WO 0113967 A
- GB 2080814 A
- WO 08070270 A
- WO 07122232 A
- EP 583170 A
- WO 9703710 A
- EP 737703 A
- EP 1779836 A
- DE 102005035879
- WO 0182886 A
- WO 9920318 A
- WO 9741900 A
- DE 60113937
- DE 4328329
- WO 0178692 A
- WO 9519795 A
- GB 2401879 A
- WO 03051412 A
- WO 9739781 A
- WO 9613285 A
- WO 03068843 A
- DE 19710369
- WO 9965538 A
- WO 0128600 A
- WO 9401468 A
- DE 4328329 C2
- DE 4028622 C2
- DE 10350654 A1


### In der Beschreibung aufgeführte Nicht-Patentliteratur


- **ZHILEI LU et al.** *Drug Delivery,* 2008, vol. 15 (2), 87-96
- CTFA-Abhandlung, Cosmetic Ingredient Handbook. The Cosmetic, Toiletry and Fragrance Association, Inc, 1988